# EUROPEAN PATENT APPLICATION

(11) **EP 4 417 251 A2**
(43) Date of publication of application: **21.08.2024**
(21) Application number: 24186323.2
(22) Date of filing: 18.12.2020
(51) Int. Cl.: A61N 5/067

(54) **SYSTEMS, METHODS AND COMPUTER-ACCESSIBLE MEDIUM FOR A FEEDBACK ANALYSIS AND/OR TREATMENT OF AT LEAST ONE PATIENT USING AN ELECTROMAGNETIC RADIATION TREATMENT DEVICE**

(30) Priority: 23.12.2019 US 201962952793 P
(62) Divisional of application: 20907047.3
(71) Applicant: Avava, Inc., Waltham MA 02451 (US)
(72) Inventor: BHAWALKAR, Jayant, Auburndale, MA 02466 (US); LEVINE, Lewis J., Marlborough, MA 01752 (US); DRESSER, Charles Holland, Wayland, MA 01778 (US); KATKAM, Rajender, Waltham, MA 02453 (US)
(74) Representative: LKGLOBAL Lorenz & Kopf Patentanwalt Attorney at Law PartG mbB

(57) **Abstract**

A system is proposed, comprising an electromagnetic radiation (EMR) treatment device configured to generate a treatment EMR beam to treat a patient tissue afflicted with a condition, the EMR treatment device being operable at a set of base parameters; at least one processor; and at least one non-transitory computer-readable storage medium storing instructions thereon that, when executed by the at least one processor, cause the at least one processor to perform a method including the steps of receive patient data from a remote network in electronic communication with the at least one processor, the patient data including at least one pretreatment image of the patient tissue; determine one or more recommended changes to the set of base treatment parameters based on the received patient data; offer the determined one or more recommended changes to a treatment operator; and append the patient data with at least one posttreatment image of the patient tissue.

## Description

### CROSS-REFERENCE TO RELATED APPLICATION(S)

This application relates to and claims priority from U.S. Patent Application Serial No. 62/952,793 filed on December 23, 2019, the entire disclosure of which is incorporated herein by reference.

### FIELD OF THE DISCLOSURE

The present disclosure relates to feedback detection and/or treatment of at least one patient, and more particularly to systems, methods and computer-accessible medium for providing feedback detection and/or treatment of at least one patient using, e.g., an electromagnetic radiation treatment/application device.

### BACKGROUND INFORMATION

Dermatological and cosmetic treatments can utilize individualized treatment parameters in order to achieve the desired effects. Particularly difficult cases involve patients with darker skin types (e.g., Fitzgerald skin type II or greater), as well as those patients with dermal pigment conditions (e.g., melasma). In order to provide individualized treatments (e.g., in difficult cases), it can be advantageous to document treatment parameters, patient data, and images of lesions before and after treatment. This information can later be used to track the progress and (where needed) modify treatment. Currently, however, this need for documentation is not streamlined, and likely requires the use of multiple system that are not enabled to communicate with one another. For example, images taken of a particular body part of the patient are typically obtained using a camera system (e.g., dermatoscope), patient data is normally stored in an electronic health record, and the treatment is performed with a separate stand-alone treatment electromagnetic radiation (EMR)-based system. For this reason, personalized tracking of specific patient outcomes and individualized treatments are available only to patients who visit the most attentive clinicians.

Melasma or *chloasma faciei* (e.g., the mask of pregnancy) is a common skin condition characterized by tan to dark gray-brown, irregular, well-demarcated macules and patches on the face. The macules are believed to be due to overproduction of melanin, which is taken up by the keratinocytes (epidermal melanosis) or deposited in the dermis (dermal melanosis, melanophages). The pigmented appearance of melasma can be aggravated by certain conditions such as pregnancy, sun exposure, certain medications (e.g., oral contraceptives), hormonal levels, and genetics. The condition can be classified as epidermal, dermal, or mixed depending on the location of excess melanin. Exemplary symptoms of melasma primarily include the dark, irregularly-shaped patches or macules, which are commonly found on the upper cheek, nose, upper lip, and forehead. These patches often develop gradually over time.

Melasma can cause considerable embarrassment and distress. It can be especially problematic for darker skin tones in women, impacting up to 30% of Southeastern Asian women, as well as many Latin American women. Only 1-in-4 to 1-in-20 affected individuals are male, depending on the population study. Approximately 6 million women in the United States are afflicted with melasma, according to the American Academy of Dermatology. Worldwide, number of people afflicted with melasma is estimated to be about 157 million in Asia/Pacific, 58 million in Latin America, and 3 million in Europe. Melasma generally appears between ages 20-40. As no cure currently exists for melasma, patients in the United States undergoing treatment for melasma currently try many different types of treatment. 79% of the United States patient's topical medications. For example, about 37% use an oral treatment, and about 25% utilize a laser.

Unlike other pigmented structures that are typically present in the epidermal region of a skin (e.g., at or near the tissue surface), dermal (or deep) melasma is often characterized by widespread presence of melanin and melanophages in portions of the underlying dermis. Accordingly, treatment of dermal melasma (e.g., lightening of the appearance of darkened pigmented regions) can be particularly challenging because of the greater difficulty in accessing and affecting such pigmented cells and structures located deeper within the skin. Accordingly, conventional skin rejuvenation treatments, such as facial peels (e.g., laser or chemical), dermabrasion, topical agents, and the like, which primarily affect the overlying epidermis (and are often the first course of treatment for melasma), may not be effective in treating dermal melasma.

Additionally, up to 50% of melasma patients also experience other hyperpigmentation problems. Among the pigmentary disorders, melasma is the one for which the largest proportion of patients are likely to visit a dermatologist. Management of this disorder remains challenging given the incomplete understanding of the pathogenesis, its chronicity, and recurrence rates. After treatment, melasma may recur, often being worse than prior to treatment. Moreover, topical treatments which may work in treating epidermal melasma can fail to effectively treat dermal or mixed melasma.

In order to successfully treat difficult conditions, such as melisma, patient outcomes should be carefully tracked and treatment parameters should be reasonably adjusted. Without feedbacks indicating treatment progression and patient responses successful treatment of melasma is only treated by the most artful clinicians. With numerous people affected by melasma and very few clinicians able to successfully treat the condition, many people afflicted with such disorder are left untreated.

It has been observed that application of light or optical energy of certain wavelengths can be strongly absorbed by pigmented cells, thereby damaging them. However, an effective treatment of dermal melasma using optical energy can introduce several obstacles. For example, pigmented cells in the dermis should be targeted with sufficient optical energy of appropriate wavelength(s) to disrupt or damage them, which may release and/or destroy some of the pigmentation and reduce the pigmented appearance. However, such energy can be absorbed by pigment (e.g., melanin) in the overlying skin tissue, such as the epidermis and upper dermis. This near-surface absorption can lead to excessive damage of the outer portion of the skin, and insufficient delivery of energy to the deeper dermis to affect the pigmented cells therein. Moreover, moderate thermal injury to melanin containing melanocytes located in the basal layer of the epidermis can trigger an increase in the production of melanin (e.g., hyperpigmentation) and severe thermal damage to the melanocytes can trigger a decrease in the production of melanin (e.g., hypopigmentation).

The Pigmentary Disorders Academy (PDA) evaluated the clinical efficacy of different types of melasma treatment in an attempt to gain a consensus opinion on an effective treatment. The findings of PDA were published in a paper entitled "Treatment of Melasma" by M. Rendon et al. published in The Journal of the American Academy of Dermatology in May of 2006. Such Rendon et al. publication reviewed literature related to melasma treatment for the 20 years prior and made determinations based upon their review. In such publication, it was stated that "[t]he consensus of the group was that first line therapy for melasma should consist of effective topical therapies, mainly fixed triple combinations," and that "[l]asers should rarely be used in the treatment of melasma and, if applied, skin type should be taken into account."

A criticism of such paper regarding melasma treatment could be that it is not very current, having been published in 2006. A more recent article by M. Sadeghpour et al. published in 2018 in Advances in Cosmetic Surgery entitled "Advances in the Treatment of Melasma" attempts to review current melasma treatment modalities. This article by Sadeghpour et al. likewise concludes that "[t]opical therapy remains the gold standard for first-line therapy for melasma using broad-spectrum sunscreens and either hydroquinone 4% cream, tretinoin, or triple-combination creams." This publication states that dermal melasma is more difficult to treat "because destruction of these melanosomes is often accompanied by significant inflammation that in turn stimulates further melanogenesis."

Therefore there is still a significant, unmet need for a more efficacious and safe treatment for melasma and other hard to treat pigmentary disorders.

Approaches have been developed that involve an application of optical energy to small, discrete treatment locations in the skin that are separated by healthy tissue to facilitate healing. Accurately targeting the treatment locations (e.g., located in dermal layer) with a desirable specificity while avoiding damage to healthy tissue around the treatment location (e.g., in the epidermal layer) can be challenging. This requires the use of, for example, an optical system with a high numerical aperture (NA) for focusing a laser beam to a treatment location. The high NA optical system delivers a sufficiently high in-focus fluence (i.e., energy density) to the dermis, while maintaining a sufficiently low out-of-focus fluence in the epidermis. U.S. Patent Application Publication No. 2016/0199132, entitled "Method and Apparatus for Treating Dermal Melasma" has indicated that this technique can be advantageous for treatment of dermal pigmentation including Melasma in research settings.

The technique described in such publication generally prefers that a focal region formed by the high NA optical system be precisely located (e.g., within a tolerance of about +/- 25µm) at a depth within a target tissue. For example, melanocytes are typically located within a basal layer of the epidermis at a depth of about 100µm from the top of the skin surface. Dermal melanophages responsible for deep melasma can be present in the upper dermis just beneath the basal layer of the epidermis (e.g., 50µm below). Therefore, a difference in the focal region depth of a few-tens of micrometers can become the difference between effectively treating dermal pigmentation and inadvertently damaging melanocytes, thereby potentially causing debilitating cosmetic results (e.g., hypopigmentation). One of the reasons for this is that an EMR-based system that effectively treats dermal pigmentation has yet to be made commercially available.

Therefore, it is desirable to provide an EMR-based treatment system that reliably locates a focal region to a prescribed depth within a tolerance of tens of micrometers (e.g., about ±100µm, about ±10µm, about ±1µm, etc.) Further, it can be desirable for such EMR-based treatment system achieve this performance in part through calibration, for example, by periodically placing the focal region at a reference having a known depth. Furthermore, it can be desirable that the reference used during calibration be used during treatment. For example, the reference can comprise an interface that establishes a robust contact with the treatment region and stabilizes the treatment region.

Thus, there may be a need to address at least some of the deficiencies described herein above.

### EXEMPLARY OBJECTS AND POTENTIAL EXEMPLARY BENEFITS

Certain developed approaches for dermal pigment treatment, like those outlined by U.S. Patent Application Publication No. 2016/0199132 can employ a selective thermionic plasma generation as a means of treatment. In these cases, laser fluence at a focal region within the dermis is above a thermionic plasma threshold (e.g., 10⁹ W/cm²), but below an optical breakdown threshold (e.g., 10¹² W/cm²). This causes a selective plasma formation when the focal region is located at a pigmented tissue (e.g., melanin) within the dermis without generating plasma in unpigmented tissue in the dermis or pigmented epidermal tissue above the focal region. The selectively formed thermionic plasma disrupts or damages the pigment and surrounding tissue. This disruption ultimately leads to clearing of the dermal pigment. Therefore, the presence of plasma during treatment within tissue being treated can be indicative of an efficacious treatment according to certain exemplary embodiments. As a parameter selection for laser-based skin treatments often depends on skin type of the patient, and indeed other individual characteristics of the patient, the presence of plasma may be used as an indication that correct treatment parameters have been achieved. This feedback can therefore be desirable for a successful treatment of various conditions, including, e.g., melisma, in populations that are generally underserved by various laser-based treatments (e.g., those with darker skin types).

Alternatively, in some cases, properties of a detected plasma may indicate that the treatment is having an adverse effect. For example, in certain exemplary situations, a transmissive window can be placed onto a skin being treated to reference the skin and keep it from moving during treatment. It is possible for treatment to fail when the laser beam etches the window. Etching of the window likely prevents a further efficient transmission of the laser to the tissue, and can often coincide with a very bright plasma formation in the window itself. If the treatment continues with an etched window, it is likely that heat accumulation within the window can cause damage to the epidermis of the skin (e.g., burning and blistering). It can therefore be advantageous, according to an exemplary embodiment of the present disclosure, to employ feedback to detect plasma formation within the window, and reduce and/or stop treatment when it occurs.

From the foregoing, it can be understood that plasma formation during treatment can be both advantageous and deleterious to treatment. Thus, systems and methods according to exemplary embodiments of the present disclosure that provide plasma detection can detect properties of the plasma and distinguish between plasma that is beneficial to tissue treatment and plasma that can be detrimental to tissue treatment continuously in real-time.

It can be desirable, according to certain exemplary embodiments of the present disclosure to image the tissue being treated from the perspective of the treatment device, and project this view onto a screen for viewing by the practitioner. In one exemplary situation, a placement of a treatment device typically occludes a practitioner's view of the tissue being treated. Thus, tissue imaging according to exemplary embodiments of the present disclosure can facilitate an accurate placement of the treatment device for targeting affected tissue. Additionally, as the goal of treatment of many pigmentary conditions is aesthetic (e.g., improve the appearance of the skin), the images of the skin can be consistently acquired under repeatable imaging conditions (e.g., lighting and distance) during imaging so that the exemplary results of treatment may be ascertained. Attempts to address some of the foregoing issues can be found in pending U.S. Patent Application Serial No. 16/447,937 entitled "Feedback Detection for a Treatment Device" by J. Bhawalkar *et al,* incorporated herein by reference in its entirety.

Additionally, successful treatment of many dermatological and cosmetic conditions require multiple treatments (often with an EMR-based device). Treatment parameters are largely patient specific and treatment progress over time can be difficult to observe. At least for these reasons, capturing, documenting, and analyzing patient and treatment data is desirable to inform ongoing treatments. However, currently no treatment platform exists that is well suited to perform these data related activities.

It has long been the hope of those suffering with pigmentary conditions, such as melasma, that an EMR-based treatment for their condition be made widely available. Accordingly, as discussed in greater detail below, an EMR-based treatment system according to exemplary embodiments of the present disclosure can be is provided that facilitates a repeatable depth positioning of the focal region within a target tissue.

One of the objects of the present disclosure is to provide a feedback and analysis system, method and computer-accessible medium that can facilitate treatment of dermatological and cosmetic condition(s), including but not limited to those that are very difficult to treat (e.g., melasma).

### SUMMARY OF EXEMPLARY EMBODIMENTS

To that end, according to certain exemplary embodiments of the present disclosure, systems, methods and computer-accessible medium can be provided to detect and record plasma events in order to document and track treatment safety and effectiveness and image the treated tissue to accurately deliver EMR to the treatment region and/or treatment of at least one patient. These capabilities can address a number of technical problems currently preventing widespread successful treatment of dermal pigmentation and other hard to treat skin conditions with EMR-based systems.

According to exemplary embodiments of the present disclosure, various systems, methods and computer-accessible medium can be provided for facilitating feedback detection and/or treatment of at least one patient. For example, it is possible to utilize a data collection system to collect data for the patient(s), and a controller to authenticate access to a remote network, aggregate the collected patient data, store the aggregated patient data on a data storage device which is in communication with the remote network, and (optionally) access a module (e.g., a service module) which can be in communication with the remote network. An electromagnetic radiation ("EMR") source can be provided that is configured to generate an EMR beam An optics configuration (e.g., focus optics) can be provided which can be configured to converge or focus the EMR beam to a focal region located (i) along an optical axis, and (ii) below a surface of a tissue of the at least one patient, and a window located at a predetermined distance away from the focal region between the focal region and the optics arrangement along the optical axis. The window can be configured to transmit the EMR beam, and contact a surface of the tissue. The optics arrangement can comprises a folded Petzval lens.

In another exemplary embodiment of the present disclosure, the module can be accessed by authenticating access to the service module. A remote network can be accessed by verifying that (i) a financial (e.g., payment) agreement is in place, (ii) a financial transaction (e.g., payment) has been received, and/or (iii) the financial transaction is pending. The remote network can be accessed by facilitating a payment of a fee, e.g., for (i) a treatment, (ii) a patient, (iii) a subscription, (iv) an image, and/or (v) a service module. The service module can include an image recognition module, a computer vision module, an electronic health record module, and/or a clinical decision making support module. The patient data can include an image of patient tissue, an age of patient, treatment session information, a patient pain score, a data collection parameter, and/or an EMR-based treatment parameter. The data collection system can be configured to collect the patient data from the tissue which is in contact with the window. Both the data collection system and the optics arrangement can be spatially registered to the window.

According to an exemplary embodiment of the present disclosure, a drug-based treatment can be performed, which can include a topical drug, an injectable drug, and/or an orally delivered drug. The electromagnetic radiation (EMR) beam can be converged to the focal region, and such convergence may be performed at a numerical aperture (NA) of 0.3 or greater. The collecting the data of the patient(s) can be performed by, e.g., illuminating the surface of the tissue, directing light from the surface of the tissue to an image plane; and sensing the light at the image plane. The data of the patient(s) can be collected by (i) inputting patient data using a user interface, or (ii) interfacing with another network facilitating device containing patient data. The data of the patient(s) can also be collected by a photoacoustic imaging, a camera, a dermatoscope subsystem, a microscope subsystem, a confocal microscope subsystem, a plasma detection subsystem, and/or a window referencing subsystem.

These and other objects, features and advantages of the exemplary embodiments of the present disclosure will become apparent upon reading the following detailed description of the exemplary embodiments of the present disclosure, when taken in conjunction with the appended claims.

### BRIEF DESCRIPTION OF THE DRAWINGS

Further objects, features and advantages of the present disclosure will become apparent from the following detailed description taken in conjunction with the accompanying Figures showing illustrative embodiments of the present disclosure, in which:
FIG. 1 is a block diagram of an apparatus for electromagnetic radiation (EMR) treatment and patient data collection, storage, and analysis, according to an exemplary embodiment of the present disclosure;
FIG. 2 is a flowchart that illustrates a method for EMR treatment and patient data collection, storage, and analysis, according to an exemplary embodiment of the present disclosure;
FIG. 3 is a block diagram of patient data storage, according to an exemplary embodiment of the present disclosure;
FIG. 4 is a block diagram of patient data analysis service modules which operate using the exemplary apparatus of FIG. 1, according to an exemplary embodiment of the present disclosure;
FIG. 5 is an illustration of an exemplary embodiment of a treatment system, according to the present disclosure;
FIG. 6 is an exemplary illustration of an EMR beam focused into a pigmented region of a dermal layer in skin, which can utilize the exemplary methods and systems according to exemplary embodiments of the present disclosure;
FIG. 7A is an exemplary absorbance spectrum graph for melanin;
FIG. 7B is an exemplary absorbance spectrum graph for hemoglobin;
FIG. 8 illustrates a graph of the absorption coefficients of melanin and venous blood and scattering coefficients of light in skin versus wavelength;
FIG. 9 is a block diagram of a treatment system, according to an exemplary embodiment of the present disclosure;
FIG. 10 is a schematic diagram of an optical system, according to an exemplary embodiment of the present disclosure;
FIG. 11 is a schematic diagram of an optical system having a microscope attachment, according to another exemplary embodiment of the present disclosure;
FIG. 12 is a schematic diagram of an optical system having a fiber coupler attachment, according to yet another exemplary embodiment of the present disclosure;
FIG. 13 is a flow diagram for effectuating an exemplary plasma detection method, according to an exemplary embodiment of the present disclosure;
FIG. 14 is a diagram of a plasma detection system, according to an exemplary embodiment of the present disclosure;
FIG. 15 is a flow diagram for implementing an exemplary window referencing procedure, according to an exemplary embodiment of the present disclosure;
FIG. 16A is a diagram of a window referencing system, according to an exemplary embodiment of the present disclosure;
FIG. 16B is an illustration of an exemplary performance of a window referencing system, according to an exemplary embodiment of the present disclosure;
FIG. 17 is a flow diagram for a method of exemplary imaging and radiation-based treatment(s), according to an exemplary embodiment of the present disclosure;
FIG. 18 is a diagram of an exemplary imaging and radiation-based treatment system, according to an exemplary embodiment of the present disclosure;
FIG. 19A is an exemplary stitched image, according to an exemplary embodiment of the present disclosure;
FIG. 19B is a flow diagram that illustrates an exemplary method for imaging stitching, according to an exemplary embodiment of the present disclosure;
FIG. 19C is an illustration of two exemplary images of tissue subjected to a keypoint detection procedure, according to some exemplary embodiments of the present disclosure;
FIG. 19D is an illustration of two exemplary images merged together highlighting inlier matching, according to some exemplary embodiments of the present disclosure;
FIG. 19E is an illustration of an exemplary unblended mosaic of stitched images, according to some exemplary embodiments of the present disclosure;
FIG. 19F is an illustration of an exemplary blended mosaic of the stitched images, according to some exemplary embodiments of the present disclosure;
FIG. 19G is an illustration of an exemplary final mosaic of the stitched images, according to some exemplary embodiments of the present disclosure;
FIG. 20 is a diagram of an exemplary apparatus for EMR treatment and visualization of treated tissue, according to an exemplary embodiment of the present disclosure;
FIG. 21 is a flow diagram of a method for EMR treatment and visualization of treated tissue, according to an exemplary embodiment of the present disclosure;
FIG. 22 is an illustration of an exemplary ray trace, according to an exemplary embodiment of the present disclosure;
FIG. 23 is an exemplary modulation transfer function (MTF) graph for a diffraction limited endoscope imaging systems according to an exemplary embodiment of the present disclosure;
FIG. 24 is an exemplary image of an exemplary configuration for an exemplary endoscope imaging system according to an exemplary embodiment of the present disclosure;
FIGS. 25A-25C are exemplary images generated using the exemplary configuration of FIG. 24;
FIG. 26 is an illustration of the exemplary ray trace according to still another exemplary embodiment of the present disclosure;
FIG. 27 is an illustration of another exemplary embodiment a data collection and treatment device/system, according to an exemplary embodiment of the present disclosure; and
FIG. 28 is an illustration of yet another exemplary embodiment of a data collection and treatment device/system, according to an exemplary embodiment of the present disclosure.

It is noted that the drawings are not necessarily to scale. The drawings are intended to depict only typical aspects of the subject matter disclosed herein, and therefore should not be considered as limiting the scope of the disclosure. The systems, devices, and methods specifically described herein and illustrated in the accompanying drawings are non-limiting exemplary embodiments and the appended claims.

### DETAILED DESCRIPTION OF EXEMPLARY EMBODIMENTS

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices, system and methods specifically described herein and illustrated in the accompanying drawings are non-limiting exemplary embodiments and that the scope of the present disclosure is defined solely by the claims which can be modified, added or otherwise, as appropriate. The exemplary features illustrated or described in connection with one exemplary embodiment may be combined with the features of other embodiments. Such exemplary modifications and variations are intended to be included within the scope of the present disclosure, and are in no way limiting any embodiment thereof.

Exemplary embodiments of the present disclosure are discussed in detail herein with respect to the exemplary treatment of pigmentary conditions of the skin, such as, e.g., melasma, to improve the appearance of such a pigmentary condition. However, the exemplary embodiments of the present disclosure can be employed or implemented for treatment of various other pigmentary and non-pigmentary conditions and/or other tissue and non-tissue targets without any limits. Examples of pigmentary conditions can include, but are not limited to, e.g., post inflammatory hyperpigmentation (PIH), dark skin surrounding eyes, dark eyes, café au lait patches, Becker's nevi, Nevus of Ota, congenital melanocytic nevi, ephelides (freckles) and lentigo. Additional examples of pigmented tissues and structures that can be treated include, but are not limited to, hemosiderin rich structures, pigmented gallstones, tattoo-containing tissues, and lutein, zeaxanthin, rhodopsin, carotenoid, biliverdin, bilirubin and hemoglobin rich structures. Examples of targets for the treatment of non-pigmented structures, tissues and conditions can include, but are not limited to, hair follicles, hair shafts, vascular lesions, infectious conditions, sebaceous glands, acne, and/or the like.

Exemplary methods or procedures for treating various skin conditions, such as for cosmetic purposes, can be carried out using the exemplary systems, devices, etc. described herein. It should be understood that, although such methods and/or procedures can be conducted by a physician, non-physicians, such as aestheticians and other suitably trained personnel may utilize the exemplary systems and/or devices described herein to treat various skin conditions with and without the supervision of a physician or another medical professional.

Further, in the present disclosure, like-named components of the exemplary embodiments generally can have similar features, and thus within a particular exemplary embodiment, each feature of each like-named component does not have to be necessarily fully elaborated upon. Additionally, to the extent that linear or circular dimensions are used in the description of the disclosed exemplary systems, devices, and methods, such dimensions are not intended to limit the types of shapes that can be used in conjunction with such systems, devices, and methods, and are certainly exemplary. A person skilled in the art would recognize that an equivalent to such linear and circular dimensions can easily be determined for any geometric shape. Sizes and shapes of the systems and devices, and the components thereof, can depend at least on the anatomy of the subject in which the systems and devices can be used, the size and shape of components with which the exemplary systems and devices would be used, and the exemplary methods and procedures in which the systems and devices will be used, and are certainly exemplary.

For example, exemplary high numerical aperture (NA) optical treatment systems are described that can focus electromagnetic radiation (EMR) (e.g., a laser beam) to a treatment region in a tissue. Unless otherwise indicated, the terms EMR, EMR beam, and laser beam are employed interchangeably herein. According to various exemplary embodiments of the present disclosure, the focused laser beam can deliver optical energy to the treatment region without harming the surrounding tissue. The delivered optical energy can, for example, disrupt pigmented chromophores and/or targets in a treatment region of the dermal layer of the skin, without affecting the surrounding regions (e.g., overlying epidermal layer, other portions of the dermal layer, and the like). In other exemplary implementations, the delivered optical energy can cause tattoo removal or alteration, or hemoglobin-related treatment.

Exemplary methods, system and devices for treating skin conditions with light or optical energy are described in U.S. Patent Application Publication No. 2016/0199132, entitled "Method and Apparatus for Treating Dermal Melasma," and in U.S. Provisional Application No. 62/438,818, entitled "Method and Apparatus for Selective Treatment of Dermal Melasma," each of which is hereby incorporated by reference herein in their entireties.

In general, exemplary systems, devices and methods are provided for treatment of pigmentary conditions in tissues. As discussed in greater detail herein, the exemplary systems, devices and methods can employ electromagnetic radiation (EMR), such as laser beams, to deliver predetermined amounts of energy to a target tissue. The EMR can be focused to a focal region and the focal region can be translated or rotated in any direction with respect to the target tissue. The predetermined amount of radiation can be configured to thermally disrupt or otherwise damage portions of the tissue exhibiting the pigmentary condition. In this manner, the predetermined amount of energy can be delivered to any position within the target tissue for treatment of the pigmentary condition such as to improve the appearance thereof.

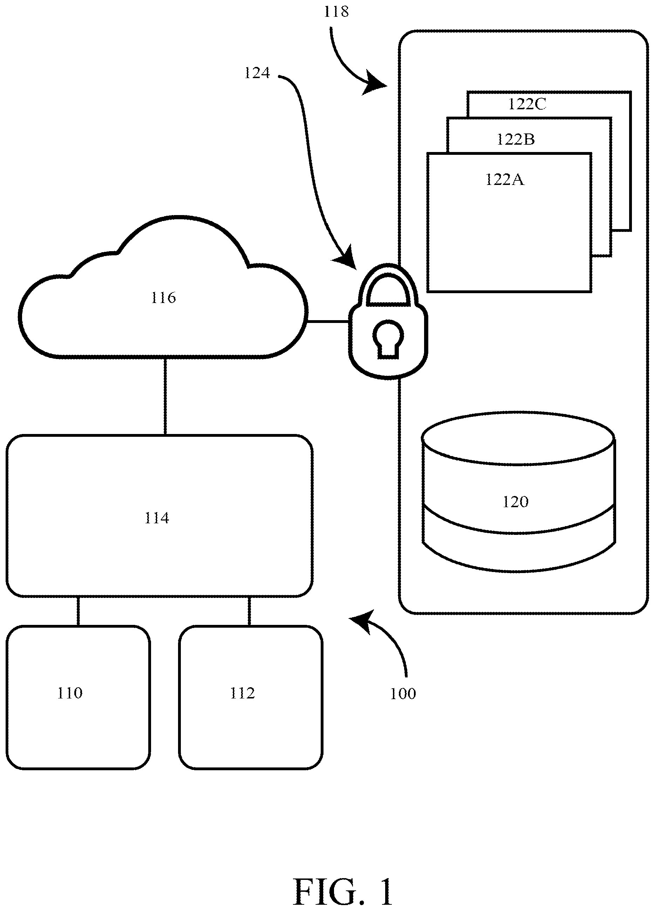

Referring now to FIG. 1, a system 100 for electromagnetic radiation (EMR) treatment and patient data collection, storage, and analysis is described and shown, according to an exemplary embodiment of the present disclosure. The exemplary system 100 illustrated in FIG. 1 can include an EMR-based treatment system 110. Exemplary EMR-based treatment systems are described in detail below, and can include a laser source (e.g., fiber laser, Q-switched Nd-YAG, diode pumped solid state [DPSS] laser, etc.) to generate a laser beam. The laser beam can be in optical communication with a focus optic (e.g., aspheric lens), which is configured to converge the laser beam at a high numerical aperture (NA) (e.g., greater than 0.2) to a focal region that is located a predetermined distance down beam (e.g., farther from the laser source generally along an optical axis in a beam path) from a window. The window can be configured to transmit the converging laser beam, and contact an outer surface of a patient's tissue, thereby positioning the focal region within the tissue (often at a predetermined depth within the tissue). The exemplary system can also include a data collection system 112. Exemplary data collection systems 112 are described in detail below. The data collection system can be configured to collect data regarding at least one of the patient, the treatment being performed, and/or the system. In some exemplary embodiments, the data collection system can comprise a sensor that senses patient data. For example, in some exemplary embodiments, the data collection system can include an illumination source configured to illuminate the surface of the tissue, an optical arrangement configured to direct light from the surface of the tissue through the window to a sensor plane, and a camera sensor configured to sense the light at the sensor plane. In other exemplary embodiments, the data collection system can collect data that is not sensed. The collected patient data can comprise a plurality of images. The aggregation of the collected patient data (e.g., by the controller) can be performed by stitching together the plurality of images. For example, in some exemplary embodiments, the data collection system can include a user interface configured to accept patient data that is manually entered by the user. In another example, the data collection system includes a system interface that is configured to accept data from another network enabled device (e.g., an electronic medical record).

Both the data collection system 112 and the treatment system 110 can communicate with a controller 114. The controller 114 can control parameters executed by the treatment system 110, as well as process data collected by the data collection system 112. In certain exemplary embodiments, the data from the data collection system 114 can be used as a basis for controlling treatment parameters of the treatment system 110. Exemplary treatment parameters can include, for example, laser pulse duration, laser power, laser pulse energy, laser repetition rate, focal region location (e.g., depth in tissue), focal region scan speed, focal region scan path, etc. The controller 114 can be connected to one or more networks 116 and/or other communications systems and/or networks. For example, the controller 114 in some exemplary embodiments can be connected to a local area network (LAN) via a network interface card (NIC). In some other exemplary embodiments, the controller 114 can be connected to a wireless local area network (WLAN) (e.g., Wi-Fi) via a wireless adapter. Such network(s) 116 can be ultimately accessible by a remote network 118. The remote network 118 can provide communication to and/or from (e.g., between) a data store 120 (e.g., one or more hard drives, memory devices, etc.) and one or more service modules 122A-122C. In an exemplary embodiment, the data store 120 can comprise non-volatile memory upon which data (e.g., patient data) can be securely stored. The service modules 122A-122C can provide resources (e.g., applications), which can be used to perform services (e.g., analyze patient data). The remote network 118 (in some exemplary embodiments) can be a virtual network, which therefore does not require the data store 120 and the one or more service modules 122A-C to be collocated.

The controller 116 can access the remote network 118 after an authentication with an access control system 124. In certain exemplary embodiments, the access control system 124 queries the controller 116 for credentials, for example, login, password, etc. The access control system 124 - in some exemplary embodiments - can provide access to the remote network 124 only after a financial process has been performed or an assurance to perform a financial process has be made. For example, access to the remote network 118 - in certain exemplary embodiments - can be granted only after a user of (or any other interested party to) the system 100 has paid a fee. The fee structure - in certain exemplary embodiments - can include one or more of the following arrangements: a fee per treatment, a fee per patient, a fee per system user, a fee per system, a fee for a subscription (i.e., fee for a time period of access), a fee for data storage, and a fee for a data module. In certain exemplary embodiments, the controller 116 can effectuate a payment of the fee electronically with a payment configurations maintained on file (either locally on the controller or remotely).

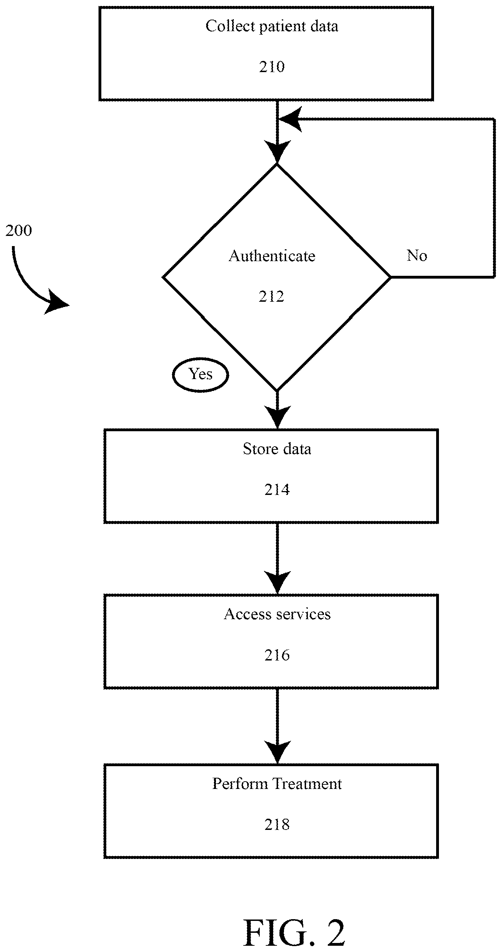

Referring now to FIG. 2, such drawings shows a flow diagram 200 of a method for electromagnetic radiation (EMR) treatment and patient data collection, storage, and analysis, according to an exemplary embodiment of the present disclosure. As illustrated in FIG. 2, data can be collected in procedure 210. The data can be related to a patient undergoing treatment. For example, patient data - in some cases - can include a digital image of patient tissue, an age of the patient, treatment session information, a patient pain score, a data collection parameter, and/or an electromagnetic radiation (EMR)-based treatment parameter. Patient data can include one or more digital images of tissue undergoing treatment, as well as other pertinent information about the patient or treatment (e.g., treatment parameters, patient feedback, etc.) In some exemplary embodiments of the present disclosure, the patient data can be aggregated for storage. Exemplary methods of data aggregation can include procedures comprising, e.g., combining data sets, stitching images, and linking data with common variables (e.g., patient ID, treatment date, etc.).

In procedure 212, access to a remote network (e.g., remote network 118) can be authenticated. Authenticating access to the remote network 212 - in some exemplary embodiments - can include an access control technique. Examples of access control techniques can include attribute-based access control (ABAC), discretionary access control (DAC), identity-based access control (IBAC), mandatory access control (MAC), organization-based access control (OrBAC), role-based access control (RBAC), and responsibility-based access control. According to some exemplary embodiments of the present disclosure, authenticating access to the remote network additionally can include verifying that (i) a payment agreement is in place, (ii) a payment has been made, and/or (iii) a payment is pending. In some exemplary embodiments, authenticating via the remote network can additionally include paying a fee. In some exemplary embodiments, paying the fee can include a payment system. Exemplary payment systems can include electronic payment systems (which can facilitate making a payment, e.g., from one bank account to another using electronic methods without direct intervention of bank employees), e-commerce payment systems (e.g., PayPal, Google Wallet, etc.), and payment systems that employ cash substitutes (such as debit cards, credit cards, electronic fund transfers, direct credits, direct debits, internet banking, and e-commerce payment systems). In some exemplary embodiments, a fee payment can be made using one of a credit card payment system, an automated teller machine (ATM) system, automated clearing house system, real-time gross settlement (RTGS) system, or a SWIFT networked system.

Once access to the remote network is attained, the exemplary method continues by storing the data in procedure 214. For example, the data can be stored to a data storage device or system (e.g., data store 120) that can be in communication with the remote network. In certain exemplary embodiments, the data storage device or system can be accessible via the remote network. Exemplary data storage devices/systems can include cloud storage, which stores the data in logical pools located across multiple servers. Data storage information can be commonly organized by patient (e.g., a unique patient identifier) in order to prevent unauthorized access of patient data. When the patient data is stored, it may be accessed by the controller as well as one or more service modules. In some exemplary cases, a picturing archiving and communication system (PACS) can be employed for physical storage and digital imaging and communications in Medicine (DICOM), which can be used as a data format for the feedback. DICOM is a standard maintained by Health Level Seven (HL7) standards group. Data associated with the feedback in some embodiments is moved into and out of the cloud. Data exchange with the remote data storage - in some exemplary cases - can be performed through the fast healthcare interoperability resources (FHIR) service, implemented by numerous vendors, for example, including Microsoft Azure cloud service and Google's Cloud Healthcare service.

In procedure 216, services can be accessed via the remote network (e.g., cloud computing). The services can be resources that are available to the controller. For example, the services can have access to and process selected data on the data storage system. In certain exemplary embodiments, the services can be processed locally on the controller. In other exemplary embodiments, the services can be processed remotely on a device (e.g., server) that can be in communication with the remote network. In still other exemplary embodiments, the services are processed, in a hybrid manner, both locally on the controller and remotely. In some exemplary cases, an individual service can use additional authentication and payment to access. Exemplary services include image recognition, computer vision, electronic health record, and clinical decision-making support, although any module supportive of treatment can be envisioned. For example, in some exemplary embodiments, a remote service can facilitate a remote user (e.g., expert clinician) to review the patient data and make comments. The remote user - in some exemplary cases - can make a diagnosis, devise a treatment plan, and/or offer valuable insights which would otherwise be unavailable to the patient.

Further, in procedure 218, an electromagnetic radiation (EMR)-based treatment can be performed. In some exemplary embodiments, the EMR-based treatment utilize collected data, remotely stored data, and/or remotely accessed services. For example, in an exemplary ongoing treatment of a pigmented lesion, a practitioner can first view images taken before and after earlier treatments, and then can titrate treatment parameters based upon the clinical images. In another example, the practitioner can access an electronic health record that can include images obtained regarding the pigmented lesion, in addition to information related to previous treatments. Technical descriptions of systems, devices and methods for EMR-based treatments according to various exemplary embodiments of the present disclosure are described in greater detail herein.

While the exemplary flow diagram of the exemplary method 200 illustrates the exemplary treatment occurring after all other steps, it is possible for treatment to occur before, during, and/or after any other step shown therein or not shown therein. For example, according to another exemplary treatment of the present disclosure, the clinician can first perform a laser treatment on a pigmented lesion, and then the clinician can collect data related to the treatment including the laser parameters and an image of the tissue post-treatment.

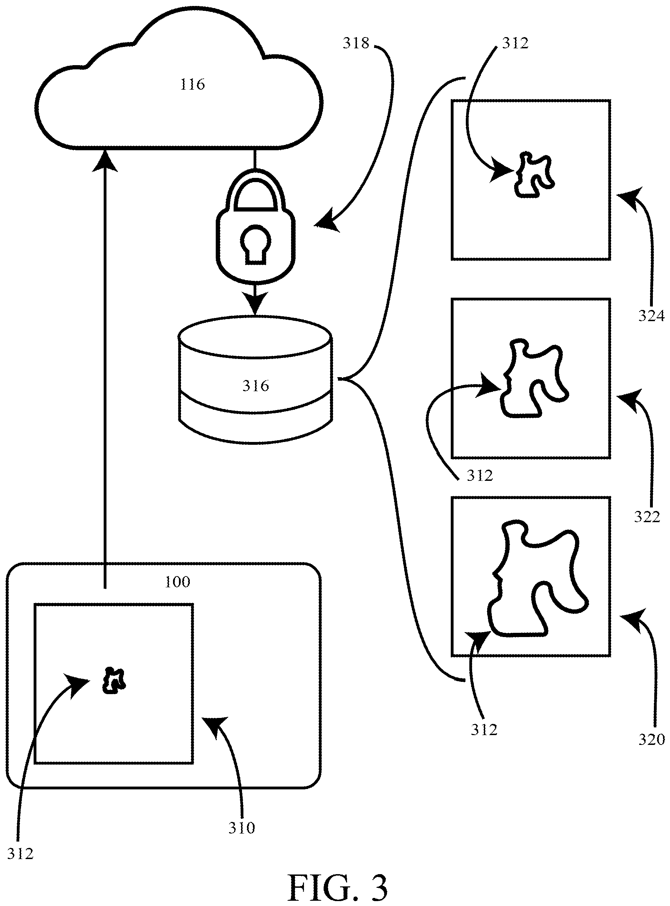

Referring now to FIG. 3, the system 100 is shown therein for storing data (e.g., digital images of tissue) remotely, according to an exemplary embodiment of the present disclosure. The system 100 is shown in FIG. 3 after capturing a most recent image 310 of a tissue having a lesion 312. The most recent image 310 can then be uploaded via one or more networks 116 to a data storage system 316. In certain exemplary embodiments, access to the data storage system 315 can be controlled by an access control system 318. Within the data storage system, the most recent image 310 can be grouped with earlier images of the same lesion 312. A first (oldest) image 320, a second (second oldest) image 322, and a third (third oldest) image 324 are all shown grouped together within the data storage system 316. Each exemplary image of the lesion was taken at a different time prior to an EMR-based treatment. For example, exemplary EMR-based treatments can be performed at intervals of a few weeks apart (e.g., 6 weeks). The pigmented lesion 312 can respond well to treatment as it is diminishing in prevalence between sessions. In certain exemplary embodiments, the stored digital data can be used to provide a record of treatment. In certain exemplary embodiments, these exemplary images can be used as constituents of an electronic medical record. In addition to the electronic medical record, any number of additional data services can be accessed through the platform, in accordance with the exemplary embodiment of the present disclosure.

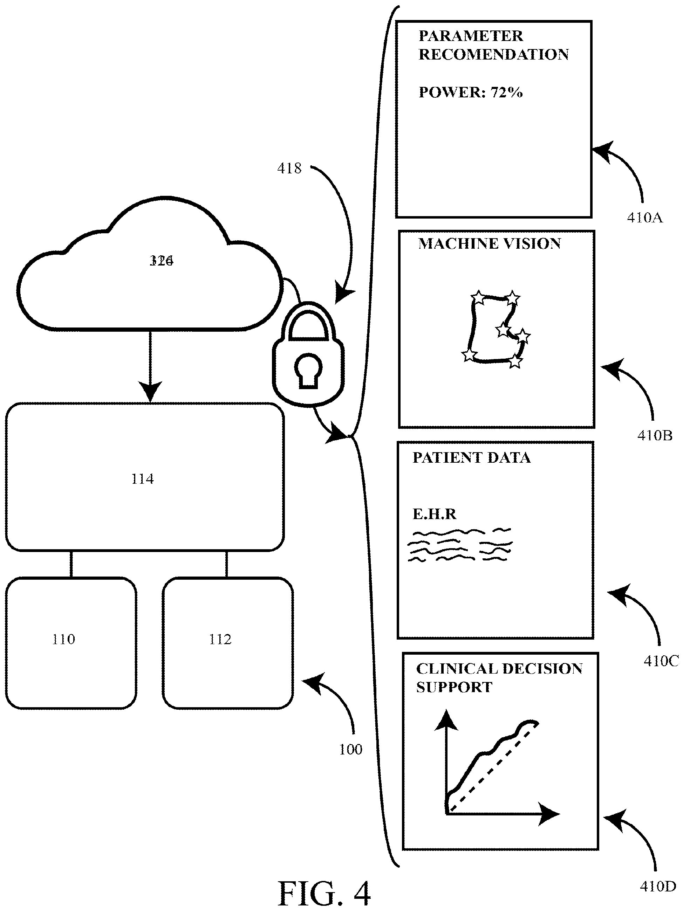

FIG. 4 illustrates the system 100 (which can be the same as or similar to the system 100 of FIG. 1) which is configured to access an array of service modules 410A-410D according to an exemplary embodiment of the present disclosure. The exemplary system 100 can access the services via one or more networks 116. In some exemplary embodiments, access to the service modules 410A-410D can be controlled using an access control system 418 (which can be the same as or similar to the access control system 118 of FIG. 1). A first service module 410A can be or include a treatment parameter recommendation application. According to some exemplary embodiments, the treatment parameter recommendation application of the first service module 410A can use one or more procedures and/or algorithms to provide recommended treatment parameters based upon selected data. For example, in some cases the treatment parameter recommendation application of the first service module 410A can receive pretreatment images of tissue that is to be treated as input, and analyze them to determine recommended treatment parameters. In certain exemplary embodiments, the treatment parameter recommendation application can use an artificial intelligence to make its recommendation. Exemplary treatment parameters that can be recommended are described below in greater detail.

A second service module 410B can be or include a machine vision module. This module can provide machine vision tools to the system 100. Exemplary machine vision resources can include, e.g., image recognition, image registration, stitching, filtering, thresholding, pixel counting, segmentation, edge detection, color analysis, blob detection, neural net/deep learning, pattern recognition, and barcode reading. The computer vision service module in some exemplary embodiments can be written using available software toolkits (e.g., OpenCV, TensorFlow, and CUDA). In some exemplary embodiments of the present disclosure, a machine vision-based service module can be used to detect a presence of a lesion on a tissue and register the lesion location with the treatment system 110. In another exemplary embodiments, a machine vision-based service module can be used to grade progression of a treatment and does so by comparing images taken before and after. In still another embodiment, a machine vision-based service module can determine from color analysis a skin type of a patient undergoing treatment.

A third service module 410C can be or include an electronic health record module. The electronic health record module can organize and provide some, most or all stored data related to an individual patient. Patients can respond differently to EMR-based treatments (e.g., a patient skin can be more or less resistant to EMR). As a result, ongoing EMR-based therapy can be used to perform individualized treatments. In order to generate a treatment plan that is custom for each individual patient, it can be important and/or beneficial for most or all pertinent patient data to be accessible to the practitioner in a single location or accessible in a single location. The electronic health record module 410B can facilitate the practitioner to access and view patient data from previous treatments (e.g., images of tissue).

A fourth service module 410C can be or include a clinical decision support module. The clinical decision support module can utilize patient data to help support clinical decisions. In certain exemplary embodiments, the exemplary clinical decision support module can predict likely outcomes of treatment. An exemplary clinical decision support system service module can calculate an area under a receiver operating characteristics curve in order to quantify a probability of a binary event occurring (e.g., a patient's pigmented lesion being successfully treated).

Although the above-indicated service modules have been described above in detail, any number of additional service modules can be employed that address the needs of the clinician, patient, or clinic administration. For example, an additional service module in some exemplary embodiments can comprise a remote access to a remote controlled which can be used by a health professional (e.g., an expert clinician) who can offer feedback on the patient data, without actually having to actually see the patient in person. Additionally, in certain exemplary embodiments of the present disclosure, EMR-based treatment is augmented with drugs (e.g., topical, oral, and injectable).

For example, exemplary systems, devices and methods for electromagnetic radiation (EMR) treatment and patient data collection, storage, and analysis according to exemplary embodiments of the present disclosure are described. Provided below is an additional description for exemplary EMR-based treatment systems 110 and data collection systems 112.

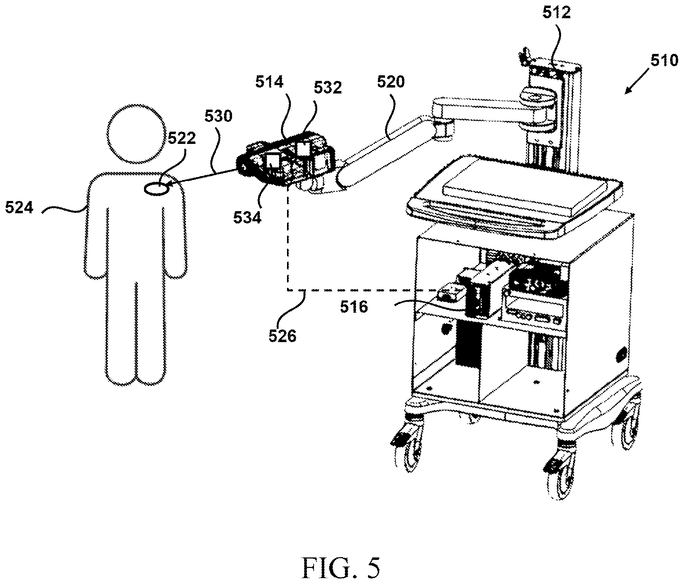

In particular, FIG. 5 illustrates an exemplary embodiment of a treatment system 510 according to another exemplary embodiment of the present disclosure. As shown in FIG. 5, the treatment system 510 can include a mounting platform 512, emitter 514, and a controller 516. The mounting platform 512 can include one or more manipulators or arms 520. The arms 520 can be coupled to the emitter 514 for performing various treatments on a target tissue 522 of a subject 524. Exemplary operation of the mounting platform 512 and emitter 514 can be directed by a user, manually or using the controller 516 (e.g., via a user interface). In certain exemplary embodiments (not shown), the exemplary emitter can have a hand-held form factor, and the mounting platform 512 can be omitted.

Emitter 514 and controller 516 (and optionally mounting platform 512) can be in communication with one another via a communications link 526, which can be any suitable type of wired and/or wireless communications link carrying any suitable type of signal (e.g., electrical, optical, infrared, etc.) according to any suitable communications protocol.

Controller 516 according to exemplary embodiment can be configured to control operation of emitter 514. In one exemplary embodiment, controller 516 can control the movement of EMR 530. As discussed in detail below, the emitter 514 can include a source 532 for emission of the EMR 530 and a scanning system 534 for manipulation of the EMR 530. As an example, scanning system 534 can be configured to focus EMR 530 to a focal region and translate and/or rotate this focal region in space. Controller 516 can send signals to source 532, via communications link 526 to command source 532 to emit EMR 530 having one or more selected properties, such as wavelength, power, repetition rate, pulse duration, pulse energy, focusing properties (e.g., focal volume, Raleigh length, etc.). In another exemplary embodiment, controller 516 can send signals to scanning system 534, via communications link 526 to command scanning system 534 to move the focal region of EMR 530 with respect the target tissue 522 in one or more translation and/or rotation operations.

Exemplary embodiments of treatment system 510 and exemplary methods are discussed herein in the context of targets within skin tissue, such as, e.g., a dermal layer. However, the exemplary embodiments can be employed for treatment of any tissue in any location of a subject, without any limitation. Examples of non-skin tissues can include, but are not limited to, surface and sub-surface regions of mucosal tissues, genital tissues, internal organ tissues, and gastrointestinal tract tissues.

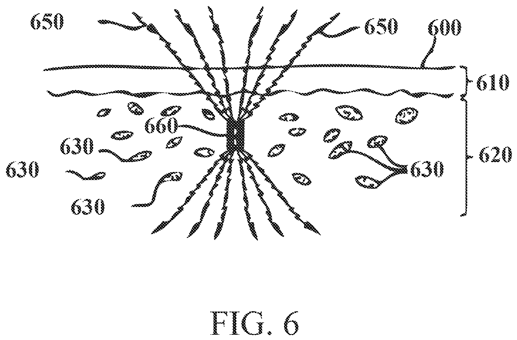

FIG. 6 shows an illustration of a laser beam focused into a pigmented region of a dermal layer in a skin tissue, using the exemplary system(s), device(s) and methods according to exemplary embodiments of the present disclosure. The skin tissue includes a skin surface 600 and an upper epidermal layer 610, or epidermis, which can be, e.g., about 30-120 µm thick in the facial region. The epidermis 610 can be slightly thicker in other parts of the body. For example, in general, the thickness of the epidermis can range from about 30 µm (e.g., on the eyelids) to about 1500 µm (e.g., on the palm of the hand or soles of the feet). Such epidermis may be thinner or thicker than the examples above in certain exemplary conditions of the skin, for example psoriasis. The underlying dermal layer 620, or dermis, extends from below the epidermis 610 to the deeper subcutaneous fat layer (not shown). Skin exhibiting deep or dermal melasma can include a population of pigmented cells or regions 630 that contain excessive amounts of melanin. Electromagnetic radiation (EMR) 650 (e.g., a laser beam) can be focused into one or more focal regions 660 that can be located within the dermis 620, or the epidermis, 610. The EMR 650 can be provided at one or more appropriate wavelengths that can be absorbed by melanin. EMR wavelength(s) can be selected based on one or more criteria described below.

### Exemplary Properties of Treatment Radiation

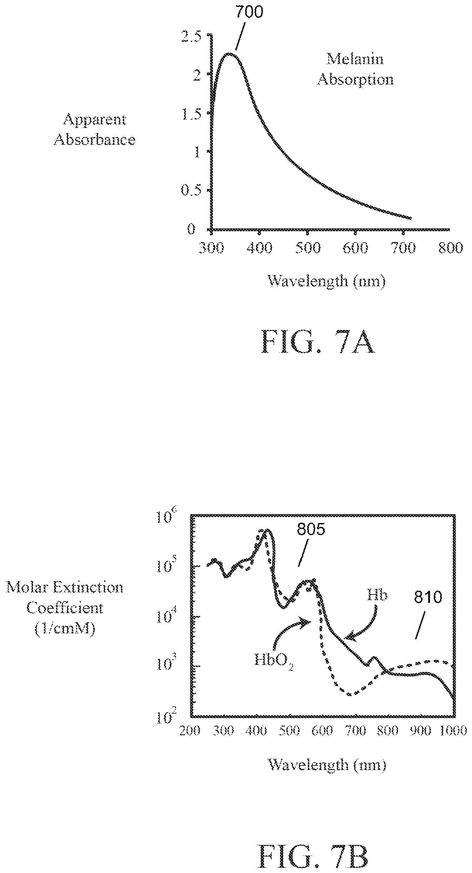

An exemplary determination of desirable wavelength for treatment of certain skin conditions, such as pigmentary conditions and non-pigmentary conditions, can depend on, for example, the wavelength dependent absorption coefficient of the various competing chromophores (e.g., chromophore, hemoglobin, tattoo ink, etc.) present in the skin. FIG. 7A shows an exemplary absorbance spectrum graph for melanin. The absorption of EMR by melanin is observed to reach a peak value 700 at a wavelength of about 350 nm, and then decreases with increasing wavelength. Although absorption of the EMR by the melanin facilitates heating and/or disruption of the melanin-containing regions 630, a very high melanin absorbance can result in a high absorption by pigment in the epidermis 610 and a reduced penetration of the EMR into the dermis 620, or the epidermis 610. As illustrated in FIG. 7A, melanin absorption is relatively high at EMR wavelengths that are less than about 500 nm. Accordingly, wavelengths less than about 500 nm may not be suitable for penetrating sufficiently into the dermis 620 to heat and damage and/or disrupt pigmented regions 630 therein. Such enhanced absorption at smaller wavelengths can result in unwanted damage to the epidermis 610 and upper (superficial) portion of the dermis 620, with relatively little unabsorbed EMR passing through the tissue into the deeper portions of the dermis 620.

FIG. 7B illustrates an exemplary absorbance spectrum graph for oxygenated or deoxygenated hemoglobin. Hemoglobin is present in blood vessels of skin tissue, and can be oxygenated (HbO₂) or deoxygenated (Hb). Each form of Hemoglobin may exhibit slightly different EMR absorption properties. As illustrated in FIG. 7B, exemplary absorption spectra for both Hb and HbO₂ can indicate a high absorption coefficient for both Hb and HbO₂ at EMR wavelengths less than about 600 nm at 805, with the absorbance decreasing significantly at higher wavelengths at 810. Strong absorption of EMR directed into the skin tissue by hemoglobin (Hb and/or HbO₂) can result in heating of the hemoglobin-containing blood vessels, resulting in unwanted damage to these vascular structures and less EMR available to be absorbed by the melanin when the desired treatment is a melanin-rich tissue or structure.

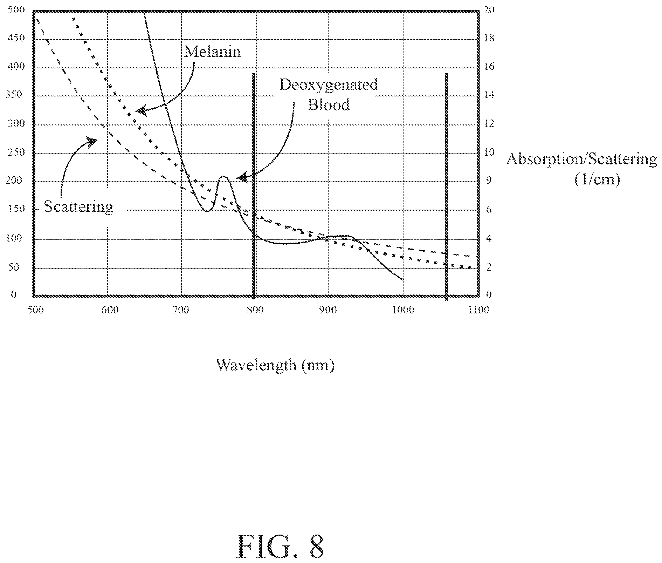

The selection of an appropriate wavelength for EMR can also depend on a wavelength dependent scattering profile of tissues interacting with the EMR. FIG. 8 illustrates an exemplary graph of the absorption coefficient of melanin and venous (deoxygenated) blood versus wavelength. FIG. 8 also shows an exemplary graph of the scattering coefficient of light in skin versus wavelength. The absorption in melanin decreases monotonically with an increase of the wavelength. If melanin is the target of a pigmentary condition treatment, a wavelength having a high absorption in melanin can be desirable. This can indicate that the shorter the wavelength of light, the more efficient the treatment can be. However, the absorption by blood increases at wavelengths shorter than about 800 nm, thereby likely increasing the risk of an unintentional targeting of blood vessels. In addition, as the intended target can be located below the skin surface, the role of scattering by skin (e.g., dermal layer) can be significant. Scattering reduces the amount of light that reaches the intended target. The scattering coefficient decreases monotonically with increasing wavelength. Therefore, while a shorter wavelength can facilitate an absorption by melanin, a longer wavelength can provide a deeper penetration due to the reduced scattering. Similarly, longer wavelengths can be more beneficial for sparing blood vessels due to a lower absorption by blood at longer wavelengths.

Based on the above considerations, wavelengths can be utilized that can range from about 400 nm to about 4000 nm, and more particularly about 500 nm to about 2500 nm, for selectively targeting certain structures (e.g., melanin) in the dermis. For example, wavelengths of about 800 nm and about 1064 nm can be useful for such treatments. The approx. 800 nm wavelength can be beneficial because laser diodes at such exemplary wavelength can be less costly and readily available to implement. Turning to approx. 1064 nm, such exemplary wavelength can be useful for targeting deeper lesions due to lower scattering at this wavelength. A wavelength of 1064 nm can also be more suitable for darker skin types in whom there is a large amount of epidermal melanin. In such individuals the higher absorption of lower wavelength EMR (e.g., about 800 nm) by melanin in the epidermis increases the likelihood of thermal injury to the skin. Hence, a wavelength of about 1064 nm may be more suitable to be used as the wavelength of the treatment radiation for certain treatments and for some individuals.

Various laser sources can be utilized for the generation and/or production of EMR. For example, Neodymium (Nd) containing laser sources are available that provide EMR at the wavelength of about 1064 nm. These laser sources can operate in, e.g., a pulsed mode with repetition rates in a range of about 1 Hz to about 100KHz. Q-Switched Nd lasers sources can provide laser pulses having a pulse duration of less than one nanosecond. Other Nd laser sources may provide pulses having pulse durations more than one millisecond. An exemplary laser source providing EMR of approx. 1060nm wavelength can be a 20W NuQ fiber laser from Nufern of East Granby, CT, USA. The 20W NuQ fiber laser can provide pulses having a pulse duration of about 100 ns at a repetition rate in the range between about 20KHz and about 100KHz. Another exemplary laser source can be an Nd:YAG Q-smart 850 from Quantel of Les Ulis, France. The Q-smart 850 can provide pulses having a pulse energy up to about 850mJ and a pulse duration of about 6 ns at a repetition rate of up to about 10 Hz.

The exemplary systems described herein can be configured to focus the EMR in a highly convergent beam. For example, the exemplary system can include a focusing and/or converging lens arrangement having a numerical aperture (NA) selected from about 0.3 to about 1 (e.g., between about 0.5 and about 0.9). The correspondingly large convergence angle of the EMR can provide a high fluence and intensity in the focal region of the lens (which can be located within the dermis) with a lower fluence in the overlying tissue above the focal region. Such focal geometry can help reduce unwanted heating and thermal damage in the overlying tissue above the pigmented dermal regions. The exemplary optical arrangement can further include a collimating lens arrangement configured to direct EMR from the emitting arrangement onto the focusing lens arrangement.

The exemplary optical treatment systems can be configured to focus the EMR to a focal region having a width or spot size that is less than about 500 µm, for example, less than about 100 µm, or even less than about 50 µm, e.g., as small as about 1 µm. For example, the spot size can have ranges from about 1 µm to about 50 µm, from about 50 µm to about 100 µm, and from about 100 µm to about 500 µm. The spot size of the focal region can be determined, for example, in air. Such spot size can be selected as a balance between being small enough to provide a high fluence or intensity of EMR in the focal region (e.g., to effectively irradiate pigmented structures in the dermis), and being large enough to facilitate the irradiation of large regions/volumes of the skin tissue in a reasonable treatment time.

A high NA optical system can deliver different energy densities to different depths along an optical axis. For example, an exemplary optical system having an NA of about 0.5 can focus a radiation to about a 2µm diameter focal region width (i.e., waist) at focus. The focal region can have a fluence (i.e., energy density) at focus of about 1J/cm². Because of the high NA (e.g., fast) optical system, at a location just 10µm out of focus the radiation has an energy density of 0.03J/cm² or 3% the energy density at focus. The radiation a mere approx. 30µm out of focus can have an energy density that is just about 0.4% (0.004J/cm²) of the in-focus energy density. This precipitous change in energy density along the optical axis can facilitate a depth selective tissue treatment to be possible; although it can also require a precise depth positioning of the focal region (e.g., to within tens of micrometers) within the target tissue.

The exemplary optical arrangement according to exemplary embodiments of the present disclosure can also be configured to direct the focal region of the EMR onto a location within the dermal tissue that is at a depth below the skin surface, such as in the depth range from about 30 µm to about 2000 µm (e.g., between about 150 µm to about 500 µm). Such exemplary depth ranges can correspond to typical observed depths of pigmented regions in skin that exhibit dermal melasma or other targets of interest. Such exemplary focal depth can correspond to a distance along the optical axis between a lower surface of the apparatus configured to contact the skin surface and the location of the focal region. Additionally, according to some exemplary embodiments of the present disclosure, the exemplary systems and methods can be configured for treating targets within the epidermis. For example, an exemplary optical arrangement may be configured to direct a focal region of the EMR to a location within the epidermis tissue (e.g., about 5 µm to about 2000 µm beneath the skin surface). According to still other exemplary embodiments of the present disclosure, the exemplary systems and methods can be configured for treating a target deep in the dermis. For example, a tattoo artist can typically calibrate the utilized tattoo gun to penetrate the skin to a depth from about 1 mm to about 2 mm beneath the skin surface. Accordingly, in certain exemplary embodiments, the exemplary optical arrangement may be configured to direct a focal region of the EMR to a location within the dermis tissue in a range from about 0.4 mm to 2 mm beneath the skin surface.

It can be desirable that an exemplary treatment system for treatment of tissues be configured to identify specific exemplary treatment areas in a target tissue. (e.g., by imaging: pigments, interface between dermal and epidermal layers in the target tissue, cell membranes, etc.). It can also be desirable to monitor/detect the interaction between the EMR and the target tissue (e.g., plasma generation in tissue). Additionally, based on the detection, the exemplary treatment system can modify the treatment process (e.g., by changing intensity, size/location of focal region in the target tissue, etc.).

Provided below are various exemplary parameters for the use with the exemplary embodiments of exemplary treatment systems according to the present disclosure

| | Min. | Nom. | Max. |
|---|---|---|---|
| Numerical Aperture | 0.01 | 0.5 | >1 |
| Depth of Focal Region (µm) | 0 | 250 | 5000 |
| Wavelength (nm) | 200 | 1060 | 20,000 |
| Rep. Rate (Hz) | 10 | 10,000 | 200,000 |
| Pulse Duration (nS) | 1×10⁻⁶ | 100 | 1×10⁵ |
| Pulse Energy (mJ) | 0.01 | 2 | 10000 |
| Average Power (W) | 0.001 | 20 | 1000 |
| M² | 1 | 1.5 | 3 |
| Laser Operation | Pulsed or Continuous Wave (CW) | | |
| Scan Width (mm) | 0.1 | 10 | 500 |
| Scan Rate (mm/S) | 0.1 | 250 | 5000 |
| No. Scan Layers (-) | 1 | 10 | 100 |
| Scan Pattern Form | Raster, Boustrophedon, Zig-Zag, Spiral, Random, etc. | | |

where depth of focal region can be a depth within the tissue (e.g., depth of focal region = 0 can be at about a surface of the tissue), and M² can be a parameter characterizing a quality of the EMR beam.

### Exemplary Feedback Detection and Exemplary EMR-Based Treatment

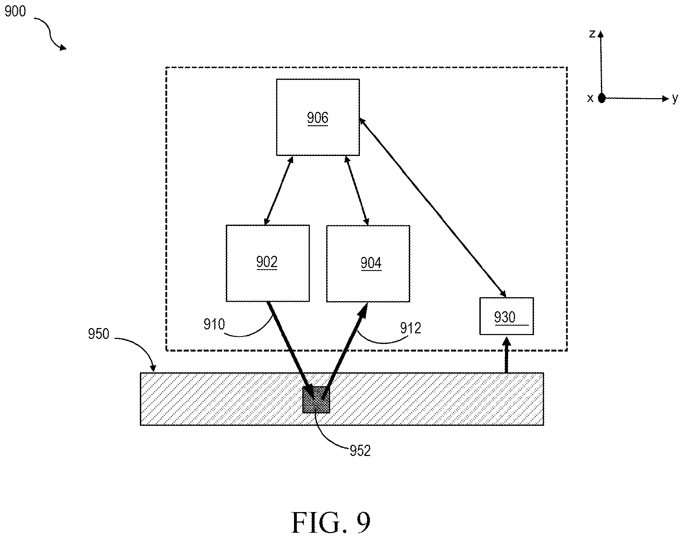

FIG. 9 shows a block diagram of an exemplary treatment system 900 according to an exemplary embodiment of the present disclosure. The exemplary treatment system 900 can include an optical system 902, an EMR detection system 904 and a controller 906 (which can include one or more computers and/or processors). The optical system 902 can include optical elements (e.g., one or more of mirrors, beam splitters, objectives, etc.) for directing EMR 910 generated by a source (e.g., a laser) to a focal region 952 of a target tissue 950. The EMR 910 can include an imaging radiation configured to image a dermal and/or epidermal layer of one or more portions of a target tissue 950 (e.g., skin). The EMR 910 can also include a treatment radiation for treatment of a region in the target tissue (e.g., region 952 of the target tissue 950). In some exemplary implementations, the EMR 910 can include only one of an imaging radiation and/or a treatment radiation in a given time period. For example, EMR 910 can include the treatment radiation for a first time duration and the imaging radiation for a second time duration. In other exemplary implementations, the EMR 910 can simultaneously include both the imaging and the treatment radiations in a given time period. According to certain exemplary embodiments, the imaging radiation can be provided at a wavelength that is, e.g., generally equal to that of the treatment radiation; and, the imaging radiation can have power that less than the treatment radiation. According to another exemplary embodiment, the imaging radiation can be provided by an imaging radiation source other than the source providing the treatment radiation, and the imaging radiation can have a wavelength different than the treatment radiation.

The EMR detection system 904 (e.g., photodiode, charged-coupled-device (CCD), spectrometer, photon multiplier tube, and the like) can detect signal radiation 912 generated, produced and/or reflected by the target tissue 950 due to its interaction with EMR 910 and/or a portion of EMR 910 reflected by the target tissue 950 being signal radiation 912. For example, EMR 910 having an intensity above a threshold value (e.g., treatment radiation) can generate a plasma in the target tissue 950. The plasma can produce the signal radiation 912, for example, due to its interaction with the EMR 910. The signal radiation 912 can be representative of properties of the plasma (e.g., the presence of plasma, the temperature of the plasma, the size of the plasma, components of the plasma, etc.)

In some exemplary implementations, EMR 910 having an intensity below the threshold value (e.g., imaging radiation) can interact with the target tissue 950 without significantly perturbing the target tissue 950 (e.g., without damaging the target tissue 950, generating plasma in the target tissue 950, etc.) The signal radiation 912 generated from such interaction can be used to image the target tissue 950 (e.g., portion of the target tissue 950 in the focal region 952 of EMR 910). This signal radiation 912 can be used to detect pigments in the target tissue 950 (e.g., pigments located in the focal region 952 of the target tissue 950). According to some exemplary embodiments, non-pigmented tissues can imaged. For example, as the imaging radiation (e.g., EMR 910) passes through cellular structures having different indices of refraction, the light is reflected as the signal radiation 912.

The exemplary optical system 902 and the exemplary EMR detection system 904 can be communicatively coupled to the exemplary controller 906. The controller 906 can vary the operating parameters of the exemplary treatment system 900 (e.g., by controlling the operation of the optical system 902). For example, the controller 906 can control the movement of the focal region 952 of the EMR 910 in the target tissue 950. As discussed in greater detail herein, this can be performed, for example, by moving the exemplary optical system 902 relative to the target tissue 950, and/or by moving optical elements within the optical system 902 (e.g., by controlling actuators coupled to the optical elements) to vary the location of the focal region 952. The controller 906 can receive data characterizing optical detection of the signal radiation 912 from the EMR detection system 904.

The controller 906 can control the properties of the EMR 910. For example, the controller 906 can instruct the source of EMR 910 (e.g., a laser source) to change the properties (e.g., intensity, repetition rate, energy per pulse, average power, etc.) of the EMR 910. In certain exemplary implementations, the controller 906 can vary the optical properties (e.g., location of focal region, beam size, etc.) of the EMR 910 by placing/controlling an optical element (e.g., objective, diffractive optical element, etc.) in the path of the EMR 910. For example, the controller 906 can place an objective in the path of EMR 910 and/or move the objective along the path of the EMR 910 to vary the size of the focal region 952 of the EMR 910.

The controller 906 can determine various characteristics of the target tissue 950 and/or interaction between the EMR 910 and the target tissue 950 (e.g., plasma generation in the target tissue 950) based on the detection of the signal radiation 912 from the EMR detection system 904. In one exemplary implementation of the exemplary treatment system 900, the controller 906 can determine one or more of a distribution of a pigment, a topography of dermal-epidermal layer junction, etc., in the target tissue 950. Furthermore, the controller 906 can be configured to generate a map indicative of the detected distribution of one or more of the exemplary properties of the target tissue 950, both described herein and those not specifically discussed. The determination of such distributions and/or generation of the distribution map can be referred to herein, but not limited to, as imaging.

In certain exemplary embodiments, the target tissue 950 can be scanned using the controller 906, that can control the EMR detection system 904 and/or the optical system 902. For example, in a Cartesian coordinate system, the target can be scanned along one or more axes (e.g., along the x-axis, the y-axis, the z-axis, or combinations thereof). In alternative embodiments, scanning can be performed according to other coordinate systems (e.g., cylindrical coordinates, spherical coordinates, etc.). The scan can be performed using the imaging beam (e.g., EMR 910 having an intensity below a threshold value) and the signal radiation 912 corresponding to various regions in the target tissue 950 in the path of the imaging beam can be detected by the EMR detection system 904. Exemplary characteristics of the signal radiation 9512 (e.g., intensity) can vary based on the pigments in the portions of the target tissue 950 that interact with the imaging beam (e.g., pigments in the focal region 952 of the imaging beam). The controller 906 can receive a signal from the EMR detection system 904 that can include data characterizing the detected characteristic (e.g., intensity) of the signal radiation 912. The controller 906 can analyze the received data (e.g., compare the received data with predetermined characteristic values of the detected signal radiation 912 in a database) to determine the presence/properties of pigments in the target tissue 950.

In some exemplary implementations, the controller 906 can determine a location of a portion of the target tissue 950 to be treated ("target treatment region") based on the signal radiation 912. For example, it may be desirable to treat a layer in the target tissue 950 (e.g., dermal layer in a skin tissue) located at a predetermined depth from the surface of the target tissue 950. The optical system 902 can be adjusted (e.g., by positioning the optical system 902 at a desirable distance from the surface of the target tissue 950) such that the focal region 952 is incident on the surface of the target tissue 950. This can be done, for example, by scanning the optical system 902 along the z-direction until the signal radiation 912 exhibits predetermined characteristics indicative of interaction between the EMR 910 and the surface of the target tissue 950. For example, an interface material (e.g., an optical slab, a gel, etc.) can be placed on the surface of the target tissue 950, and as the focal region 952 transitions from the target tissue 950 to the interface material, the characteristic of the signal radiation 912 can change. This can be indicative of the location of the focal region 952 of the EMR 910 at or near the surface of the tissue. Once the optical system 902 is positioned such that the focal region 952 of the EMR 910 is at or near the surface of the target tissue 950, the optical system 902 can be translated (e.g., along the z-direction) such that the focal region 952 is at the predetermined depth below the surface of the target tissue 950.

The controller 906 can vary the operating parameters of the exemplary treatment system 900 based on the signal received from the EMR detection system 904 including data characterizing the detected characteristic of the signal radiation 912. For example, some exemplary embodiments of the EMR detection system 904 can detect a depth of a dermis-epidermis (DE) junction in the target tissue 950, and the controller 906 can adjust a depth of the focal region 952 in response to the depth of the DE junction. In this exemplary manner, the DE junction can be employed as a reference for determining the depth of the focal region 952 within the dermis. Additionally, in some exemplary embodiments, the EMR detection system 940 can quantify a proportion of melanin present in an epidermal layer of a skin (e.g., via use of a spectrophotometer). Based upon the proportion of melanin, the controller 906 can provide the ability to implement one or more changes in laser parameters to a designated personnel (e.g., a clinician). According to certain exemplary embodiments, the changes in laser parameters can include, e.g., varying energy per pulse inversely with the proportion of melanin detected, increasing focus angle with an increase in the proportion of melanin, and/or modifying depth of the focal region 952 based upon the proportion of melanin.

In some exemplary implementations, an acoustic sensor 930 can be coupled to the target tissue 950, and the acoustic sensor 930 can detect characteristics of interaction between the EMR 910 and the target tissue 950. For example, an acoustic sensor can detect pressure waves, e.g., at or in the focal region 952 generated by the creation of plasma in the target tissue 950 (e.g., plasma generated in focal region 952). Examples of the acoustic sensor 930 can include, e.g., piezoelectric transducer(s), capacitive transducer(s), ultrasonic transducer(s), Fabry-Perot interferometer(s), and/or piezo electric film(s).

In one exemplary aspect, the pressure waves in the focal region 952 can be or include shock waves, a sharp change in pressure propagating through a medium (e.g., air) at a velocity faster than the speed of sound in that medium. In another exemplary aspect, the pressure waves, e.g., at the focal region 952 can be acoustic waves that propagate through the medium at a velocity about equal to the speed of sound in that medium.

Photoacoustic imaging (optoacoustic imaging) is a biomedical imaging modality based on the photoacoustic effect. In the photoacoustic imaging, e.g., non-ionizing laser pulses are delivered into biological tissues (when radio frequency pulses are used, the technology is referred to as thermoacoustic imaging). Some of the delivered energy can be absorbed and converted into heat, leading to transient thermoelastic expansion and thus wideband (i.e. MHz) ultrasonic emission.

Sensor measurement data from the acoustic sensor 930 can be transmitted to the controller 906. The controller 906 can use this data for validation of pigment detection via the signal radiation 912. According to some exemplary embodiments, the treatment can be confirmed through the detection of the shock waves. The presence and/or the intensity of pressure waves can be correlated to a plasma being generated and a plasma mediated treatment being performed. Additionally, by mapping at which the pressure waves, e.g., at or in focal regions 952 are detected, a comprehensive map of treated tissue may be created and documented.

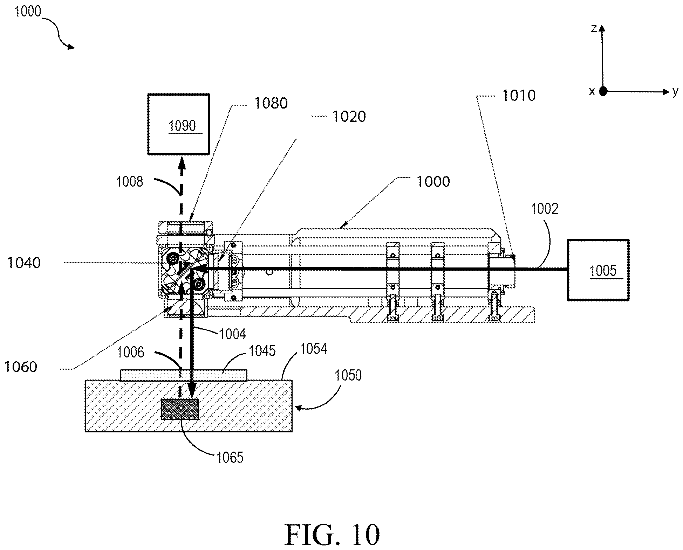

FIG. 10 shows a diagram of another exemplary embodiment of an optical system 10600. For example, the optical system 1000 can guide the EMR beam 1002 from an EMR source 1005 to a target tissue 1050. The EMR source 1005 can be a laser (e.g., a Q-smart 450 laser from Quantel that has a 450mJ pulse energy, a 6 nanosecond [nS] pulse duration, and a wavelength of 1064 nm or harmonic of approx. 1064 nm). According to certain exemplary embodiments, the EMR beam 1002 can be introduced into the exemplary optical system 1000 via an adapter 1010. The adapter can be configured to secure an EMR source that generates the EMR beam 1002 to an articulating arm e.g., arm 520 of the mounting platform 512 of FIG. 5.

According to certain exemplary embodiments, a diffractive optical element (DOE) 1020 (e.g., beam splitters, multi-focus optics, etc.) can be placed in the path of the EMR beam 1002. The DOE 1020 can alter the properties of the EMR beam 1002, and transmit a second EMR beam 1004. For example, the DOE 1020 can generate multiple sub-beams that are focused to different focal regions. Implementations and use of the DOE 1020 for treatment of target tissue are discussed in greater detail in U.S. Provisional Application 62/656,639, entitled "Diffractive Optics For EMR-Based Tissue Treatment," the entire disclosure of which is incorporated by reference herein. The second EMR beam 1004 (e.g., multiple sub-beams) generated and/or transmitted by the DOE 1020 can be directed toward the target tissue 1050 by a beam splitter 10640 (e.g., a dichroic beam splitter). An example of a dichroic beam splitter can include a short pass dichroic mirror/ beam splitter that has a cutoff wavelength of about 950 nm, a transmission band between about 420nm to about 900nm, and a reflection band between about 990 to about 1600nm (Thorlabs PN DMSP950R). The second EMR beam 1004 can be reflected by the beam splitter 10640, and directed to an objective 1060. The objective 1060 can focus the second EMR beam 1004 to a focal region 1052 in the target tissue 1050 via a window 1045. An example of the objective 1062 can be or include an Edmunds Optics PN 67-259 aspheric lens having a diameter of about 25 millimeters (mm), a numerical aperture (NA) of about 0.83, a near infrared (NIR) coating, and an effective focal length of about 15 mm. The window 1045 can be used to hold or otherwise maintain the target tissue 1050 in place.

In certain exemplary implementations, the EMR beams 1002, 1004 can be expanded by a beam expander (not shown) placed in the path of the EMR beams 1002, 1004. Beam expansion can allow for a desirable NA value of the optical system 1000. For example, a laser beam generated by a Q-smart 450 laser can have a beam diameter of about 6.5 mm, and can utilize a beam expander that can expand the laser beam to twice the diameter. The expanded EMR beams 1002, 1004 can be focused using an approximately 15 mm EFL lens to focus the EMR beams 1002, 1004 with a sufficiently high NA (e.g., greater than 0.3).

The exemplary optical system 1000 can be arranged and/or configured such that the focal region 1052 of the second EMR beam 1004 can be located below the epidermis of the target tissue 1050. This can be done, for example, by moving the exemplary optical system 1000 relative to the target tissue 1050 and/or moving the objective 1060 along the beam path of the second EMR 1004. In one exemplary implementation, a position of the exemplary optical system 1000 and/or of the exemplary optical elements in the optical system 1000 can be moved by the exemplary controller 905 of FIG. 9. Placing the focal region 1052 below the epidermis (e.g., below the dermis-epidermis (DE) junction) can reduce or substantially inhibit undesirable heat generation in the epidermis, which can lead to hyperpigmentation or hypopigmentation of the epidermis. This can also allow for targeting of regions in the dermis for heat and/or plasma generation.

Interaction between the second EMR beam 1004 and the target tissue 1050 can lead to the generation of the signal radiation 1006. As described above, the signal radiation 1006 can include radiation generated by plasma in the target tissue 1050 ("tissue radiation"). The tissue radiation 1050 can have wavelengths that lie in the transmission band of the beam splitter 1040. As a result, tissue radiation can be largely transmitted by the beam splitter 10640. The signal radiation 1006 can also include radiation having a wavelength that is similar to that of the second EMR beam 1004 ("system radiation"). The wavelength of the system radiation 1004 can lie in the reflection band of the beam splitter 1040. As a result, a small portion (e.g., 10%) of the system radiation can be transmitted by the beam splitter 1040.

The signal radiation 1008 transmitted by the beam splitter 1040 can include both the tissue radiation and the system radiation 1004 (or a portion thereof). Portions of the signal radiation 1008 can be captured by EMR detector 1090. The EMR detector 1090 can communicate data characterizing the detection of the signal radiation 1008 (or a portion thereof) to the controller 906 of FIG. 9. The controller 906 can, for example, can perform the detection (e.g., intensity of the transmitted signal radiation 1008) and at also, e.g., alter the operation of the source 1005 (e.g., switch off the source 1005).

In one exemplary implementation, the exemplary optical system 1000 can be used as a confocal microscope. This can be done, for example, by placing a second objective (not shown) upstream from the aperture 1080. The aperture can reimage the signal radiation 1006 by focusing at a focal plane that includes the aperture 1080. The aperture 1080 can filter (e.g., block) undesirable spatial frequencies of the signal radiation 1008. This exemplary configuration can facilitate filtering of the signal radiation 1008 associated with different regions in the target tissue 1050 (e.g., regions of target tissue at different depths relative to tissue surface 1054). By changing the distance between the imaging aperture 1080 and the target tissue 1050 (e.g., by moving the imaging aperture 1080 along the path of the signal radiation 1008), different depths of the target tissue 1050 can be imaged. In certain exemplary implementations, the controller 906 of FIG. 9 can move the imaging aperture 1080 by transmitting commands to an actuator. The controller 906 can analyze the detection data, and/or determine the presence of plasma in the target tissue 1050, distribution of pigments in the target tissue, and the like. The exemplary optical system 1000 can be used to detect damage in the window 1045. The damage to the window 1045 can be caused by interaction between the second EMR beam 1004 and the window 1045 (e.g., when the intensity of the EMR beam is high, prolonged interaction with the second EMR beam 1004, etc.). The detection of the damage in the window 1045 can be implemented by determining a change in the intensity in the signal radiation 1006 (e.g., emanating from the window 1045) resulting from damage in the window 1045. This can be done, for example, by positioning the focal region 1052 incident on the window 1045 (e.g., near the surface of the window 1045, at the surface of the window 1045, within the window 1045), and detecting the intensity of the signal radiation 1006 (e.g., by using a photodetector as the EMR detector 1090). This intensity can be compared with an intensity previously measured when the focal region 1052 is located on comparable location of an undamaged window 1045. Based on this comparison damage in the window 1045 can be determined.

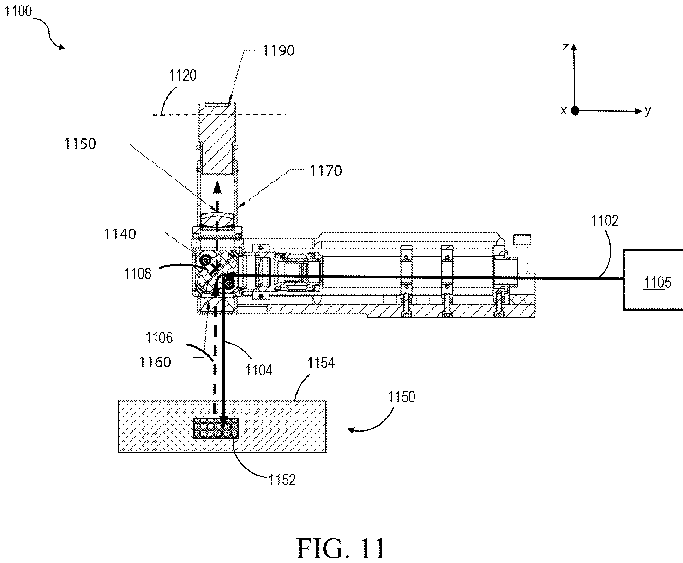

FIG. 11 provides a diagram of an exemplary embodiment of an exemplary optical system 1100. The optical system 1100 can include a microscope attachment 1170 having an eyepiece 1190. The microscope attachment 1170 can capture the signal radiation 1008 (or a portion thereof) transmitted by the beam splitter 1040 of FIG. 10. The signal radiation 1008 can be reimaged by a tube lens 1150 (e.g., Edmunds Optics PN 49-665 25 mm Diameter × 50 mm EFL aspherized achromatic lens). The tube lens 1150 can reimage the signal radiation 1008 to a pupil plane 1120 of the eyepiece 1190 (e.g., Edmunds Optics PN 35-689 10X DIN eyepiece).

As described herein, the signal radiation 1008/1108 can include both tissue radiation and system radiation. Due to difference in their wavelengths, images of the tissue radiation and system radiation are generated at different locations (e.g., at different planes). As a result, if the eyepiece 1190 is positioned to capture the image generated by system radiation, it may not be able to accurately capture the image associated with tissue radiation. However, the eyepiece 1190 can be calibrated to capture signal radiation having a different wavelength than the system radiation at the focal region of the system radiation. One exemplary way of calibrating the eyepiece 1190 can be by using a material having an index of refraction similar to that of the target tissue 1050/1150 as a phantom (e.g., acrylic). Calibrating the eyepiece 1190 can include, e.g., focusing the second EMR (beam) 1004/1104 into the phantom (e.g., by objective 1060/1160) and inducing a breakdown (e.g., laser induced optical breakdown) at the focal region of the second EMR beam 1004/1104. This can be followed by impinging the second EMR radiation 1004 having a predetermined wavelength onto the phantom (e.g. at an oblique angle), and measuring the intensity of EMR radiation having the predetermined wavelength at the eyepiece 1190. The axial location of the eyepiece 1190 can be adjusted (e.g., along the z-axis) to increase and/or maximize the intensity of detected radiation from a second EMR source. In certain exemplary embodiments, a sensor can be used instead of the eyepiece 1190. Examples of such exemplary sensors can include, e.g., CMOS and/or CCD imagers. The sensor(s) can generate a digital image in response to the radiation at a sensor plane. The digital image can represent an image of the focal region 1052/1152.

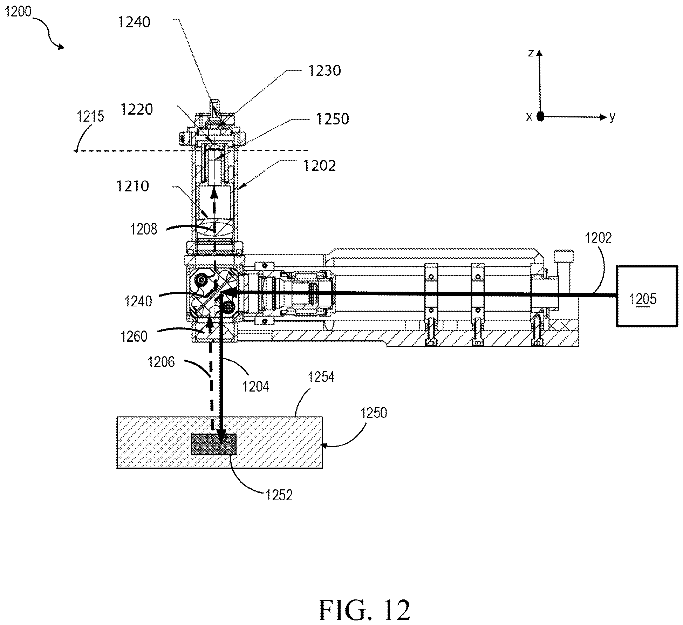

FIG. 12 illustrates another exemplary embodiment of an exemplary optical system 1200 having a fiber coupler attachment 1202. The fiber coupler attachment 1202 can include a lens tube 1210 that can image light from the objective 1060 and the beam splitter 1040 of FIG. 10 as described herein. The lens tube 1210 can focus the signal radiation 1008/1208 at a pupil plane 1215 (e.g., plane parallel to the x-y axis and including the collimating lens 1220). The focused signal radiation 1008/1208 can be collimated to a desirable size using the collimating lens 1220, and can be directed to a coupling lens 1230. The coupling lens 1230 can focus the signal radiation 1008/1208 with an NA which can be beneficial for coupling into a fiber attached to a fiber connector 1240. The fiber can be optically connected to one or more EMR detectors (e.g., the detector 904 of FIG. 9). According to certain exemplary embodiments, the coupler attachment 1202 can further comprise an imaging aperture 1250 located at the pupil plane 1215. The aperture 1250 can filter portions of the signal radiation 1008 that are not emanating from the focal region 1052/1252. According to certain exemplary embodiments, a detection instrument (e.g., photodiode, spectrometer, etc.) may be placed directly after the imaging aperture 1250 without a fiber optic or related optics. Calibration of the imaging aperture 1250 relative the lens tube 1210 may be achieved in a process similar to that described above in reference to calibration of the eyepiece 1190 of FIG. 11.

Exemplary feedback detection can be used in conjunction with EMR-based treatment in many ways. Exemplary applications are described herein to demonstrate some ways feedback informed EMR-treatment may be practiced. Broadly speaking, the examples described below may be categorized into three species of feedback informed EMR-treatment. These exemplary species can encompass examples that a) detect plasma, b) reference a focal region position; and/or c) image a tissue. Such exemplary categories of use are not intended to be an exhaustive (or mutually exclusive) list of applications for feedback informed EMR-based treatment.

### Exemplary Plasma Feedback Examples

Some exemplary treatments can include the formation of a plasma during treatment (e.g., thermionic plasma or optical breakdown). In certain exemplary embodiments, properties of a detected plasma are indicative of potential effectiveness of treatment. For example, in treating a dermal pigment condition a focal region is located deep within the skin, so that it will coincide with dermal pigment as it is scanned during treatment. As the focal region is scanned over the skin, a laser source delivers a pulsed laser, such that where the focal region and dermal pigment coincide thermionic plasma is formed. The exemplary formation of the thermionic plasma is indicative that a) a pigment is present within the skin, b) the pigment at a moment of plasma formation is collocated with the focal region (e.g., X-Y coordinates, as well as depth), and/or c) the pigment at this location has been treated (e.g., the pigment has been disrupted).

In other exemplary situations, the plasma formation can indicate a need/preference for system maintenance. For example, some systems can include a window that is placed in contact with a tissue undergoing treatment. The window can serve many functions including: contact cooling, stabilizing the tissue, providing a depth reference for the tissue, and evacuating blood or other fluids from the tissue through pressure. Radiation (e.g., laser beam) also passes through the window for application to a treatment region below. In some exemplary cases, the radiation can cause breakdown within the window or at a surface of the window, resulting in plasma generation and window etching. If the system continues to deliver radiation after plasma generation at the window, burning or thermal damage of the tissue directly in contact with the window often results.

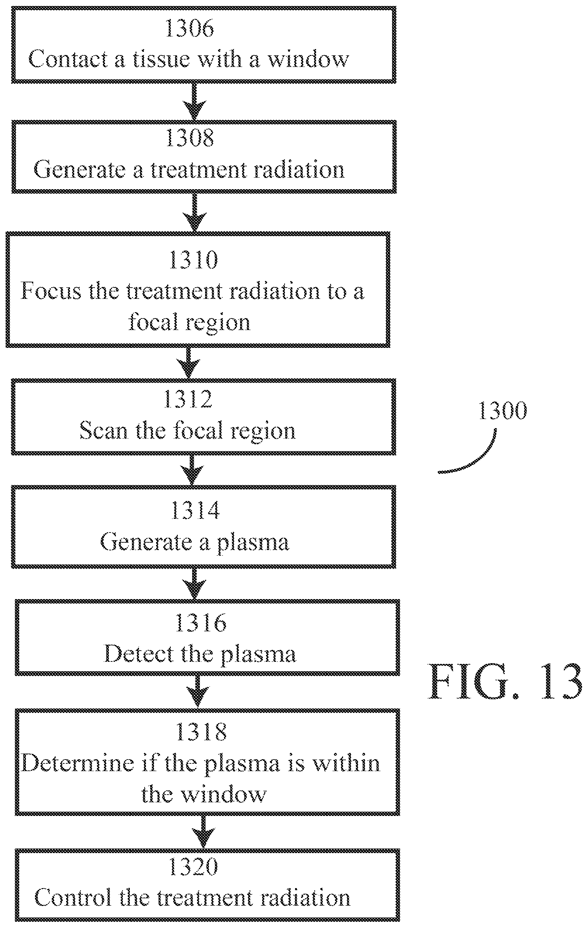

FIG. 13 illustrates a flow diagram of a plasma detection method 1300 during a radiation-based tissue treatment, according to certain exemplary embodiments of the present disclosure. First, a surface of a tissue is contacted using a window in step 1306. The window contacts an outer surface of the tissue. The window is configured to transmit a treatment radiation. For example, the window can provide a datum surface, such that placing the surface of the tissue in contact with the window effectively references the outer surface of the tissue. According to certain exemplary embodiments, the window can provide and/or facilitate the performance of additional functions including, but not limited to, preventing movement of the tissue during treatment, contact cooling of the tissue being treated, evacuation of blood (or other competing chromophores) within the tissue through compression, etc.

A treatment radiation can then be generated in step 1308. The treatment radiation can typically be generated by a radiation source. The treatment radiation can be configured to produce an effect in the tissue, which can result in an improved or desired change in appearance. In certain exemplary embodiments, tissue effects can be cosmetic. In other exemplary embodiments, tissue effects can be therapeutic. According to certain exemplary embodiments of the present disclosure, the tissue effect can include a generation of selective thermionic plasma in presence of a chromophore. Exemplary parameter selection for a treatment radiation can be dependent on the treatment being performed as well as the tissue type and individual patient. Exemplary details related to treatment radiation generation of the exemplary method 1300 and relevant parameter selection to produce an effect in tissue (e.g., a cosmetic effect) are described in detail herein.

The treatment radiation can be focused to a focal region in step 1310. For example, in step 1310, the treatment radiation can be focused by a focus optic. According to certain exemplary embodiments, the focal region can have a width that is smaller than about 1mm, about 0.1mm, about 0.01mm, or about 0.001mm. The focal region may be positioned at a first region. In certain exemplary embodiments, the first region can be located within the tissue specifically at a location to be treated. In some exemplary cases, the first region can be intentionally or unintentionally located outside of the tissue, for example, within the window that is in contact with the tissue.

The focal region can be scanned in step 1312, typically by a scanning system (e.g., scanner). Examples of the exemplary scanning procedure can include tipping/tilting the focal region, rotating the focal region, and/or translating the focal region. Further description of exemplary relevant scanning procedures and systems is provided in U.S. Patent Application Serial No. 16/219,809 entitled "Electromagnetic Radiation Beam Scanning System and Method," to Dresser et al., the entire disclosure of which is incorporated herein by reference. According to certain exemplary embodiments, the treatment radiation can be pulsed, such that approximately no treatment radiation is delivered as the focal region can be scanned (e.g., moved for the first region to a second region). The focal region may also be scanned continuously. In this exemplary case, different configurations of the timing of treatment radiation pulses and scan parameters control the locations for the first region and the second region can be implemented.

As shown in FIG. 13, plasma can be generated by the treatment radiation in step 1314. The plasma can typically be generated within or near the focal region, because fluence is at a maximum within the focal region. According to certain exemplary embodiments, plasma can be generated in step 1314 selectively a pigmented region through thermionic-plasma generation. Alternatively, the plasma may be generated in procedure 1314 through a non-selective laser induced optical breakdown.

Further, the plasma can then be detected in step 1316. For example, a detector can detect the signal radiation emanating from the plasma in such procedure 1316. Examples of the signal radiation detection can include optical detection, acoustic detection, spectroscopic detection of laser induced breakdown (e.g., laser induced breakdown spectroscopy), plasma generated shockwave (PGSW) detection, plasma luminescence detection, plasma (plume) shielding detection, and plasma photography. In certain exemplary embodiments, properties of the plasma are determined based upon the detection of the plasma in procedure 1316. Certain examples of properties of the plasma can include presence of plasma, intensity of plasma, spectral content of plasma, and position of plasma. According to certain exemplary embodiments, a property of the signal radiation can be recorded and stored, for example by the controller (e.g., a computer processor).

In certain exemplary embodiments, in procedure 1318, it can be determined if the plasma is located at least partially within the window, e.g., based upon the detected plasma. For example, in certain exemplary embodiments, an optical signal radiation comprising a spectral component known to be representative of a material in the window (and not in the tissue) may be detected indicating that the plasma is partially within the window. In another version, intensity of an optical signal radiation may reach exceed a known threshold implying that the plasma is at least partially within the window.

In step 1320, exemplary parameters related to the treatment radiation can be controlled based in part upon the detected plasma (e.g., the determination of step 1318 that the plasma is or is not partially located in the window). Examples of parameters related to the treatment radiation can include, but are not limited to, an energy per pulse, a repetition rate, a position of the focal region, or a size of the focal region. These exemplary treatment radiation parameters can be employed alone or in combination with one another or other treatment radiation parameters without limit. For example, the determination that the plasma is partially located in the window may be used as a triggering event to cease the treatment radiation.

In certain exemplary embodiments, an exemplary map can be generated that comprises a matrix of properties mapped to location, for example by the controller. As an example, the map can include a first property of a first signal radiation emanating from a first plasma at a first location can be mapped to a coordinate for the first location, and a second property of a second signal radiation emanating from a second plasma at second location mapped to a coordinate for the second location. An exemplary map can include, e.g., a four-dimensional matrix having three orthogonal axes related to the position of the focal region, and a fourth axes related to one or more properties of the plasma. In some versions, the map may be used as an indication of individual treatment effectiveness. An exemplary system suitable for performing the above described plasma detection method is described in detail herein.

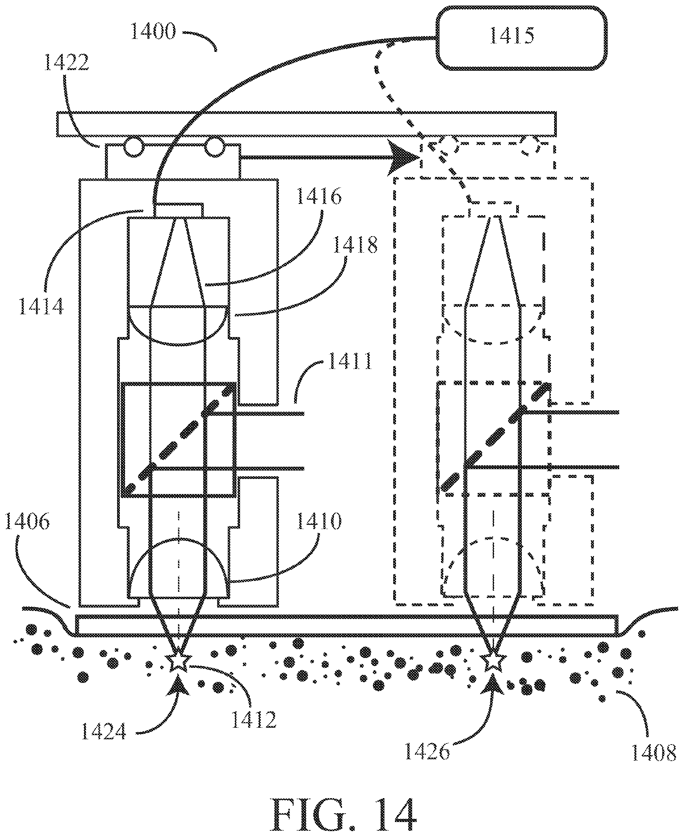

In particular, FIG. 14 shows a diagram of a plasma detection and treatment system 1400, according to certain exemplary embodiments of the present disclosure. For example, a window 1406 can be configured to contact a surface of a tissue 1408, for example - an outer surface of the tissue 1408. The window 1406 can include an optical material configured to transmit the EMR beam, for example: glass, a transparent polymer (e.g., polycarbonate), quartz, sapphire, diamond, zinc-selenide, or zinc-sulfide.

The exemplary imaging and treatment system 1400 of FIG. 14 can include a focus optic 1410. The focus optic 1410 (e.g., an objective) can be configured to focus an electromagnetic radiation (EMR) beam 1411, and generate plasma 1412 within the tissue 1408. The plasma 1412 can be generated selectively at a chromophore within the tissue 1408 through thermionic generation. In other exemplary embodiments, the plasma 1412 can be non-selectively generated through optical breakdown. The EMR beam 1411 may be generated using a radiation source (not shown). The EMR beam 1411 can comprise any of collimated or non-collimated light and coherent and non-coherent light.

A detector 1414 can be provided in the exemplary system 1400 which is configured to detect the plasma 1412. Examples of such detector(s) 1414 can include photosensors, for example, photodiodes and image sensors; acoustic sensors, for examples surface acoustic wave sensors, piezoelectric films, vibrometers, and etalons; and, more specialized detectors, for example spectrometers, spectrophotometers, and plasma luminance (or shielding) optical probes.

As shown in the drawings (including FIG. 14), the plasma detector can comprises a photodetector (e.g., a photodiode) which (in one exemplary embodiment) can be oriented toward the window 1406, which can sense visible light 1416 (e.g., signal radiation) emanating from the plasma 1412. According to certain exemplary embodiments, a tube lens 1418 can be used in conjunction with the focus optic 1410 to direct and focus the visible light 1416 incident on the detector 1414. The detector 1414 can be in communication with a controller 1415, such that data associated with the detected plasma is input to the controller 1415.

A scanner 1422 of the exemplary system of FIG. 14 can be configured to scan a focal region of the EMR beam 1411. The scanner 1422 can scan the focal region in at least one dimension. In certain exemplary embodiments, the scanner 1422 can scan the focal region in, e.g., all three dimensions. Referring to FIG. 14, the scanner 1422 is provided which can, as shown therein, scan the focal region left to right from a first region 1424 to a second region 1426 of the tissue 1408. As the scanner 1422 scans the focal region, the EMR beam 1411 can be pulsed, causing a first plasma to be generated at the first region 1424 and then a second plasma to be generated at the second region 1426. The first plasma 1412 and the second plasma 1426 can both be detected by the detector 1414. In certain exemplary embodiments, data associated with the first detected plasma and the second detected plasma are input to the controller 1415. In certain exemplary embodiments, the data associated with one or more plasma events are used by the controller 1415 to control parameters associated with at least one of the EMR beam 1411 and the scanner 1422.

According to certain exemplary embodiments, the controller 1415 can be configured to control the EMR beam 1411 (e.g., terminate the EMR beam 1411) based upon a determination if the first plasma 1412 is located at least partially within the window 1408. In one example, the controller 1415 can determine if the first plasma 1412 is at least partially located within the window 1406 based upon an intensity of the signal radiation 1416 emanating from the plasma 1412. The intensity of the signal radiation 1416 may be detected using a photosensor (e.g., photodiode). According to another version, the controller 1415 can determine if the plasma 1412 is at least partially located within the window 1406 based upon a spectral component of the signal radiation 1416. For example, according to certain exemplary embodiments, the window 1406 can comprise sapphire, which comprises aluminum. A spectra peak corresponding to aluminum is centered at about 396nm. Skin does not normally contain aluminum. Therefore, if the signal radiation (taken a precise time after a laser pulse [e.g., 10µs]) comprises a spectral peak centered at about 396nm it is likely that the first plasma 1412 is at least partially located within the window 1406. According to certain exemplary embodiments, a spectral filter (e.g., notch filter) and a photosensor is used to detect the spectral content of the signal radiation. According to other exemplary embodiments, a spectrometer or spectrophotometer is used to detect the spectral content of the signal radiation.

The controller 1415 can be configured to record one or more detected properties of the plasma 1412. In certain exemplary embodiments, the controller 1415 can be configured to record a matrix (or map) of detected properties of the plasma 1412. For example, the controller 1415 may be configured to: record a first property of a first signal radiation emanating from the first plasma 1412 at a first location 1424; map the first property to a coordinate for the first location 11024; record a second property of a second signal radiation emanating from a second plasma at a second location 1426; and map the second property to a coordinate for the second location 1426.

### Exemplary Focal Depth Referencing Examples

As described in detail herein above, a depth of a focal region within a tissue needs to be tightly controlled (e.g., +/- 20µm), in certain exemplary embodiments. For example, the treatment of dermal pigment can require a focal region be placed at a depth approximately at the depth of the dermal pigment within the tissue. If the focal region is too deep below the dermal pigment treatment would not be effective. If the focal region is too shallow, melanocytes at the basal layer will be irradiated potentially causing an adverse event (e.g., hyperpigmentation or hypopigmentation).

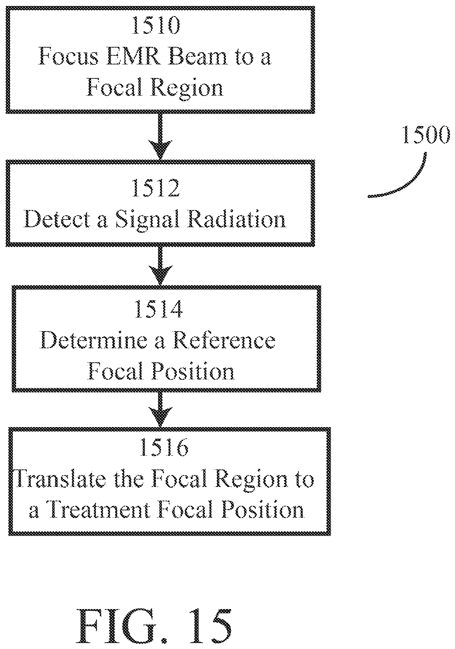

FIG. 15 shows a flow diagram of a focal depth referencing method 1500, according to certain exemplary embodiments of the present disclosure. First, in procedure 1510, an electromagnetic radiation (EMR) beam can be focused along an optical axis to a focal region. In many cases, the EMR beam can be generated by an EMR source (e.g., laser). An optical window can be disposed to intersect the optical axis. In some exemplary embodiments, a surface of the window can be substantially orthogonal to the optical axis. The EMR beam can impinge upon at least one surface of the optical window and a signal radiation can be generated. The signal radiation in certain exemplary embodiments comprises a reflected portion of the EMR beam that can be reflected at a surface of the window. In certain exemplary embodiments, the window can be configured to contact a tissue. The surface of the window can be understood optically as an optical interface between a window material of the window and an adjacent material proximal the surface of the window (e.g., air or tissue). According to various exemplary embodiments, a difference in an index of refraction between the window material and the adjacent material can result in reflection of the reflected portion of the EMR beam. According to certain exemplary embodiments, a signal radiation can be generated by scatter or transmission of a portion of the EMR beam at the window.

Turning back to FIG. 15, the signal radiation can be detected in procedure 1512. According to certain exemplary embodiments, the signal radiation can be imaged by an imaging system. In some cases, an image of the signal radiation is formed at a sensor by the imaging system. Examples of sensors can include photosensors and image sensors. In some exemplary versions, a detector detects and measures an image width. In general, the image width will be proportionally related to a beam width of the EMR beam incident the surface of the window. A magnification of the imaging system typically determines the proportionality of the image width to a width of the EMR beam incident the window. According to certain exemplary embodiments, the detector can detect and/or measure an intensity of the signal radiation.

Based upon the signal radiation, a reference focal position can be determined in procedure 1514. For example, in some exemplary embodiments, the beam width of the EMR beam incident a surface of the window is measured, and a focal position of the focal region is translated along the optical axis as the beam width is measured. The reference position is found where the beam width is determined to be at a minimum. For another example, in some exemplary versions, an intensity of the signal radiation is detected as the focal position of the focal region is translated along the optical axis. In this exemplary case, the reference position can be found where a radiation signal intensity is found to be at a maximum.

Once the reference focal position is determined, the focal region can be translated to a treatment focal position in procedure 1516. For example, the treatment focal position can be a predetermined distance away from the reference focal position along the optical axis. According to certain exemplary embodiments, the focal region can be translated by moving an optical element (e.g., objective) along the optical axis. In other exemplary embodiments, the focal region can be translated by adjusting a divergence of the EMR beam, for example adjusting an optical power of an optical element. Eventually, the window is placed in contact with a target tissue resulting in the focal region being positioned within the target tissue. According to certain exemplary embodiments, the target tissue can be skin and the focal region can be positioned within a dermal tissue of the skin. A precise depth positioning of the focal region within tissue can facilitate a treatment of previously untreatable pigmentary conditions through thermionic-plasma or thermal disruption. For example, the EMR beam can perform selective thermionic-plasma mediated treatment of dermal pigmentary condition (e.g., dermal melasma) at a focal region located within the dermis without risking adverse irradiation of the epidermis.

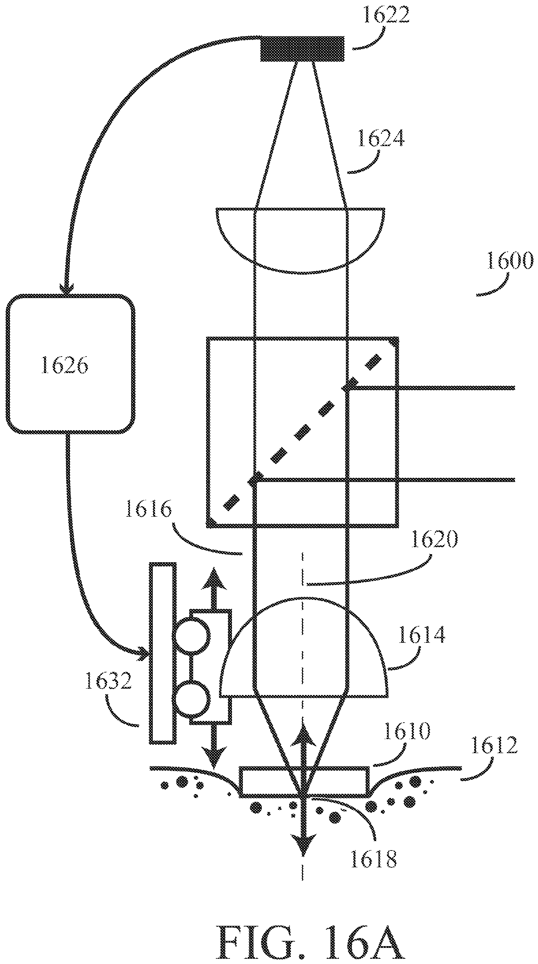

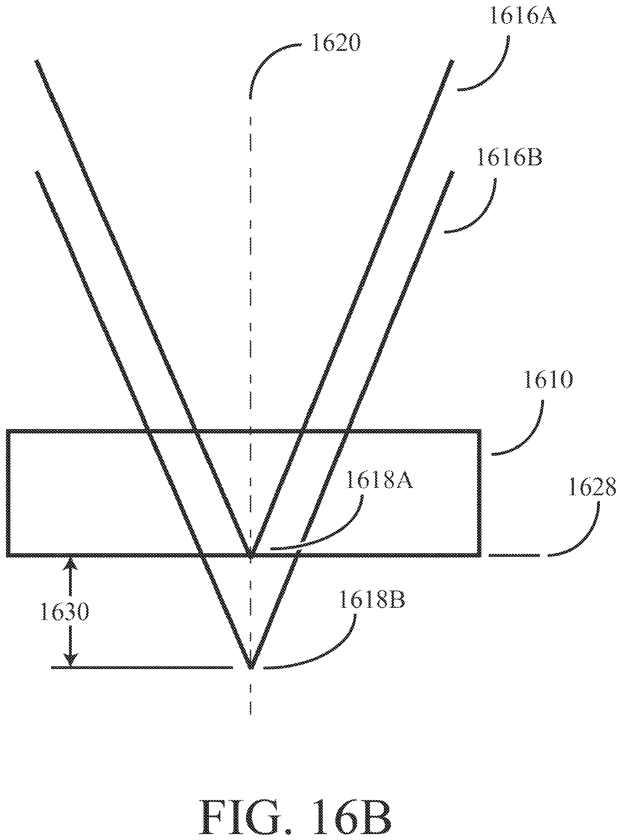

FIGS. 16A and 16B shows diagrams of a focal depth referencing and treatment system 1600 and the exemplary method, according to certain exemplary embodiments of the present disclosure.

For example, referring to FIG. 16B, a first EMR beam 1616A may be configured (only) for referencing, e.g., by bringing a first focal region 1618A incident upon the surface of window 11810 and a second EMR beam 1616B may configured to achieve the desired effect in the tissue (e.g., a cosmetic effect). Indeed, the second EMR beam 1616B can be configured to be converged by the focus optic to the second focal region 1618B located in the treatment position. This may be advantageous in various exemplary embodiments, where the tissue effect can require a very high fluence (e.g., 10¹²W/cm²) and a window 1610 would likely be damaged if the first EMR beam were to be used during referencing. According to certain exemplary embodiments, the second EMR beam 1616B can have a wavelength that approximately equal to the first EMR beam 1616A. In other exemplary embodiments, the second EMR beam 11816B can have a wavelength that is different than that of the first EMR beam 1616A. In this exemplary case, the treatment position may require calibration based upon differences in a focal length of the focus optic at such different exemplary wavelengths.

The exemplary focal depth referencing system 1600 shown in FIG. 16A includes a window 1610 configured to contact a target tissue 1612. The exemplary optical system (e.g., objective or focus optic) can be configured to focus an electromagnetic radiation (EMR) beam 1616 to a focal region 1618 along an optical axis 11820. The optical axis 11820 intersects the window 11810. An optical detector 1622 can be configured to detect a signal radiation 1624. According to certain exemplary embodiments, the signal radiation 1624 can be generated by an interaction between the EMR beam 1620 and the window 1610. In some exemplary embodiments, the interaction between the EMR beam 1620 and the window 1610 can be an interaction between a surface of the window 1610 and the EMR beam 1620. The interaction between the EMR beam 1620 and the window 1610 typically is at least one of reflection, transmission, and scatter.

A controller 1626 can be configured to take input from the optical detector 1622, and translate a focal position of the focal region 1618 along the optical axis 1620. Based at least in part upon feedback from the optical detector 1622, the controller 1626 can determine a reference position 1628, where a portion of the focal region 1618 can be substantially coincident with a surface of the window 1610. The signal radiation 1624 can emanate from a reflection of the EMR beam 1616 incident the surface of the window 1610 and be imaged incident an image sensor 1622 using (in part) the focus optic 1614. According to certain exemplary embodiments, the controller 1626 can determine the reference position by, e.g., determining a transverse width of the EMR beam 1616 that is incident upon the surface of the window based upon the signal radiation; and translating the focal region until the transverse width has a minimum value. According to another exemplary embodiment, the signal radiation emanates from a reflection of the EMR beam 1616 at a surface of the window 1610 and the detector 1622 can be configured to detect an intensity of the signal radiation. In this exemplary case, the controller 1626 can determine the reference position by translating focal region until the intensity of the signal radiation 1624 has a maximum value.

Further, the controller 1626 can translate the focal region 1618 to a treatment position a predetermined distance 1630 from the reference position 1628. In general, translating the focal region 1618 away from the reference position 1628 can be performed in a positive direction along the optical axis 1620 (i.e., away from the optical system 1614). In certain exemplary embodiments, the treatment position can be configured to be located within a tissue. For example, the predetermined distance can be configured to locate the treatment position within a dermal tissue in skin. A stage 1632 can be used to translate one or more optical elements (e.g., the focus optic) in order to translate the focal region. The EMR beam 1616 can be configured to perform an effect in tissue (e.g., a cosmetic effect) at or near the focal region located in the treatment position. An example tissue effect is selective thermionic plasma-mediated treatment of the tissue 1612.

In certain exemplary embodiments, a second EMR beam can be configured to be converged by the focus optic to a second focal region located in the treatment position. In this exemplary case, the first EMR beam may be configured only for referencing and the second EMR beam may configured to perform the tissue effect. This may be advantageous in embodiments, where the tissue effect requires, e.g., very high fluence (e.g., 10¹²W/cm²) and the window 1610 would likely be damaged during referencing. According to certain exemplary embodiments, the second EMR beam can have a wavelength that is identical to the first EMR beam. In other embodiments, the second EMR beam has a wavelength that is different than that of the first EMR beam. In this case, the treatment position will need to be calibrated based upon differences in a focal length of the focus optic at the two different wavelengths. In certain exemplary embodiments, the exemplary window referencing and treatment system 1600 can be used to measure more than one reference position 1628.

For example, according to certain exemplary embodiments, the exemplary window referencing and treatment system 1600 can also include a scanning system. The scanning system can be configured to move the focal region 1618 and the optical axis 1620 in at least one scan axis. In some exemplary cases, the scan axes can be generally perpendicular to the optical axis 1620. A parallelism measurement between the window and a scan axis can be determined by way of multiple reference position 1628 measurements at multiple scan locations. For example, the exemplary referencing and treatment system 1600 can be first used to determine a first reference position at a first scan location. Then, the scanning system can relocate the optical axis 1618 to a second scan location a distance along the scan axis from the first scan location. The exemplary referencing and treatment system 1600 can then determine a second reference position. A difference between the first and second reference positions divided by the distance along the scan axis can indicate a slope of non-parallelism between the window and the scan axis. Individual embodiments are provided below to further explain focal depth referencing in an EMR treatment device.

### Tissue Imaging Examples

An exemplary EMR-based treatment informed by tissue imaging feedback can have wide-ranging uses and benefits for dermatologic and aesthetic treatments. For example, according to certain exemplary embodiments, tissue imaging allows the user to accurately target a treatment site during EMR-based treatment. Another exemplary use of tissue imaging can be to provide documentation of treatment results overtime (e.g., pre-treatment images and post-treatment images). According to still other exemplary embodiments, tissue imaging is used to ascertain a diagnosis or a treatment plan for a condition prior to treatment, or an endpoint during a treatment. The goal of many exemplary EMR-based skin treatments is aesthetic (e.g., relating to the appearance of the skin). In these exemplary cases, imaging of the skin undergoing treatment provides some of the most important feedback to treatment stakeholders (patients and practitioners).

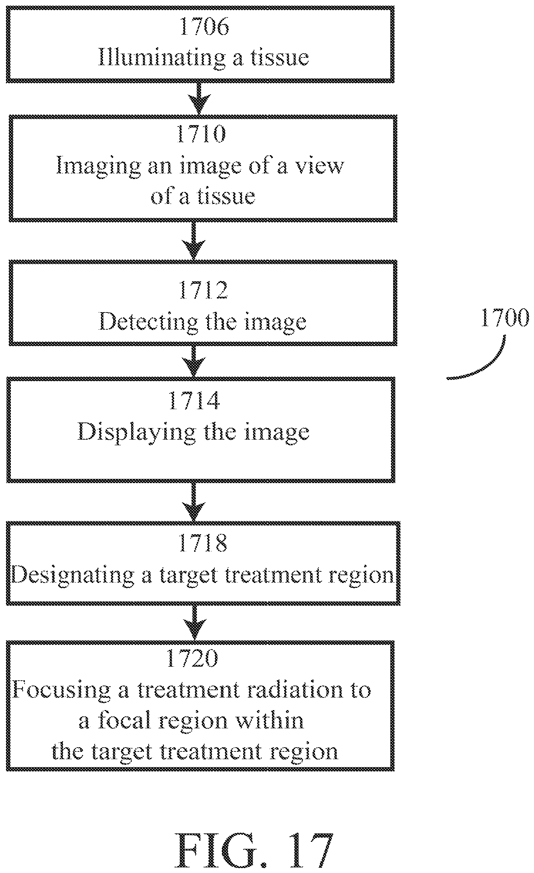

FIG. 17 illustrates a flow diagram for a method 1700 of imaging and radiation-based treatment, according to certain exemplary embodiments of the present disclosure. In the exemplary method 1700, a tissue is illuminated with an imaging radiation in procedure 1706. For example, the illumination of the tissue can be achieved at least in part by using an illumination source. The example illumination may be performed in a number ways including, e.g., bright-field illumination, where the imaging radiation is provided substantially on-axis to an imaging system and dark-field illumination, where the imaging radiation is provided substantially off-axis to the imaging system. In certain exemplary embodiments, the imaging radiation can be substantially monochromatic. In other exemplary embodiments, the imaging radiation can be substantially broadband (e.g., white light).

Further, in procedure 1710, an image of a view of the tissue can be imaged. For example, imaging can at least partially be performed using a focus optic (e.g., objective). The view in some cases can be a field of view of a focal region associated with the focus optic. In certain exemplary embodiments, the imaging procedure 1710 can include the use of one more additional optics in conjunction with the focus optic. For example, the focus optic may significantly collimate light from the view and a tube lens may be used to form the image from the collimated light. The image may be formed at an image plane.

In procedure 1712, the image can be detected. For example, a detector can be used to detect the image. Examples of the detection can include, e.g., photodetection, confocal photodetection, interferometric detection, and spectroscopic detection. The detector may detect the image at the image plane. The image may be detected by an image sensor. Examples of image sensors include semiconductor charge-coupled devices (CCD), active pixel sensors in complementary metal-oxide-semiconductor (CMOS), and N-type metal-oxides-semiconductor (NMOS). Image sensors can output a detected image in a two-dimensional (2D) matrix of data (e.g., bitmap).

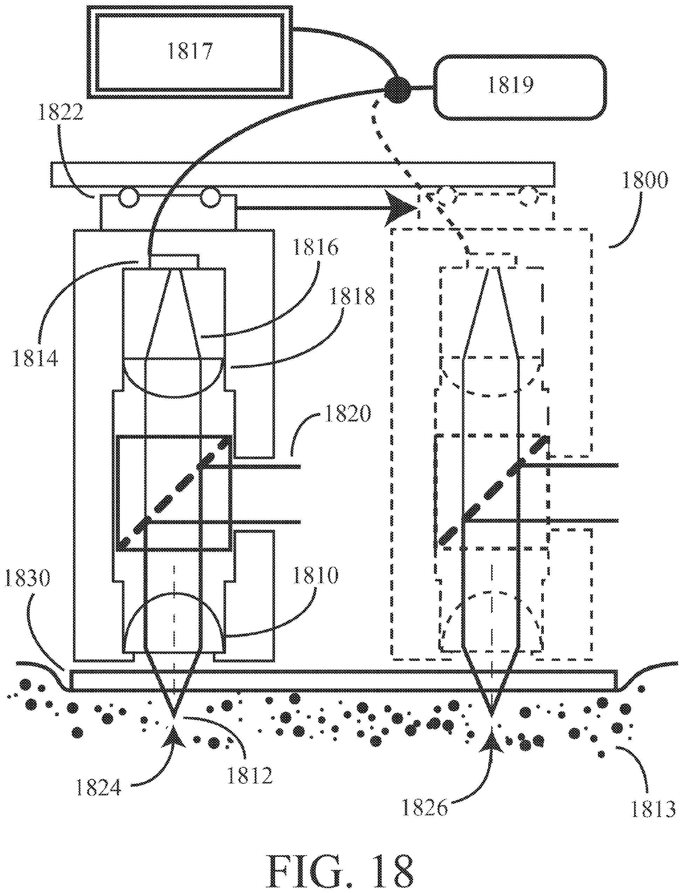

Additionally, in procedure 1714, the image can be displayed. For example, the image can be displayed by an electronic visual display. Examples of displays can include, e.g., electroluminescent (EL) displays, liquid crystal (LC) displays, light-emitting diode (LED)-backlit liquid crystal (LC) displays, light-emitting diode (LED) displays (e.g., organic LED (OLED) displays, and active-matrix organic LED (AMOLED) displays), plasma displays, and quantum dot displays. The displayed image can be viewed by a designated user (e.g., clinician). In some exemplary cases, the image can be recorded and stored, for example, by the controller 1819 of FIG. 18 or another controller as described herein. According, to certain exemplary embodiments the displayed image can be used to target a region of tissue needing treatment.

A target treatment region can then be designated within the tissue in procedure 1716. In certain exemplary embodiments, the target treatment region can be designated based in part on the image. For example, the target treatment region may be designated 1716 based upon an apparent excess of pigment (e.g., dermal melanin) in a portion of the tissue as displayed in the image. In some cases, a clinician viewing the displayed image designates the target treatment region. Alternatively, in certain exemplary embodiments, the controller can automatically designate the target treatment region based upon the image. The target treatment region is typically at least partially present in the image.

Finally, a treatment radiation can be focused to a focal region within the treatment region in procedure 1718. Typically, the treatment radiation is focused using the focus optic and configured to perform an effect within the tissue (e.g., selectively generate thermionic plasma at a chromophore; achieve a cosmetic effect). In certain exemplary embodiments, parameters affecting the treatment radiation are controlled based in part upon the image. Parameters affecting treatment with the treatment radiation are described in detail above. In certain exemplary embodiments, the focal region is scanned within the target treatment region

In certain exemplary embodiments, the view is scanned from a first region to a second region of the tissue. Examples of scanning include: tipping/tilting the view, rotating the view, and translating the view. Further description of a scanning configuration is described in U.S. Patent Application Serial No. 16/219,809 "Electromagnetic Radiation Beam Scanning System and Method," to Dresser et al., the entire disclosure of which incorporated herein by reference. In certain exemplary embodiments, the view located at the first region overlaps with the view located at the second region. In this case some of the tissue is present in both the first region and the second region. In some other embodiments, the view located at the first region does not overlap with the view located at the second region. In certain exemplary embodiments, scanning of the view can be achieved with feedback related to the view position. For example, in some exemplary cases, the view can be scanned by moving the focus optic with two linear stages. Feedback from encoders present on each linear stage may be used to infer the position of the view when located at the first region and/or the second region.

A second image may be imaged of the view from the second region. For example, imaging the second image can be performed in the same manner as imaging the first image of procedure 1710, only the location of the view is different between the two steps. Imaging is at least partially performed using the focus optic. The view in some cases can be the field of view of the focal region associated with the focus optic. The second image may be detected. Typically, detecting the second image is performed in the same manner as detecting the first image of procedure 1712, the only difference being the second image is detected instead of the first image.

In some exemplary cases, the first image and the second image are stitched together into a stitched image (or map). The stitched image may also include additional images taken with the view located at additional regions. The stitched image may be used to document a pre-treatment image of the tissue, or a post-treatment image of the tissue. Any of the first image the second image, and the stitched image may be taken prior to treatment and used to support a determination of a diagnosis, for example by a medical professional. Likewise, any of the first image, the second image, and the stitched image may be taken during or after treatment to demonstrate effectiveness of treatment or to look for *end-points* during treatment, which can suggest treatment be ended.

FIG. 18 shows a diagram of an exemplary tissue imaging and treatment system 1800, according to certain exemplary embodiments of the present disclosure. The exemplary imaging and treatment system 1800 can include a focus optic 1810. The focus optic 1810 (e.g., objective) can be configured to image a view 1812 of a tissue 1813. A detector 1814 can be configured to detect an image 1816 formed at least in part by the focus optic 1810. The detector 1814 can be in communication with a display 1817. The display is configured to display the image to a designated user (e.g., clinician). According to certain exemplary embodiments, a tube lens 1818 can be used in conjunction with the focus optic 1810 to form the image 1816. The detector 1814 can be in communication with a controller 1819, such that data associated with the detected image from the detector can be input to the controller 1819. The focus optic 110 is used for delivery of a treatment radiation 1820 as well as imaging. A scanner 1822 can be configured to scan the view 1812. The scanner can scan the view in at least one dimension, and likely in more dimensions. In certain exemplary embodiments, the scanner 1822 can scan the view in all three dimensions. Referring to FIG. 18, the scanner 1822 is shown as, e.g., scanning the view 1812 from a first region 1824 to a second region 1826 of the tissue 1813.

As the scanner 1822 scans the view 1812, the focus optic 1810 can image a first image at the first region 1824 and a second image at the second region 1826. The first image and the second image cane both be detected by the detector 1814. Further, e.g., data associated with the first detected image and the second detected image can be input to the controller 1819. In certain exemplary embodiments, the data associated with multiple images can be stitched together by the controller 1819, yielding a stitched image (or map). The stitched image and/or one or more images can be recorded and stored by the controller for future viewing. In certain exemplary embodiments, data from one or more images can be used to determine a treatment region. According to certain exemplary embodiments, determining the treatment region can be performed automatically by the controller. In other exemplary embodiments, the determination of the treatment region can be performed manually by the designated user after viewing one or more images.

The treatment radiation 1820 can be focused to a focal region by the focus optic 1810. Further, the focal region can be directed to the treatment region. According to certain exemplary embodiments, the scanner 1822 can be configured to scan the focal region within the treatment region. Certain exemplary embodiments of the exemplary system 1800 can include a window 1830 that can be placed in contact with a surface of the tissue 1813. The window 1830 can serve several purposes, one being to datum an outer surface of the tissue. The window 1830 can therefore facilitate the focal region to be reliably located within the tissue 1813 a predetermined depth from the surface of the tissue 1813.

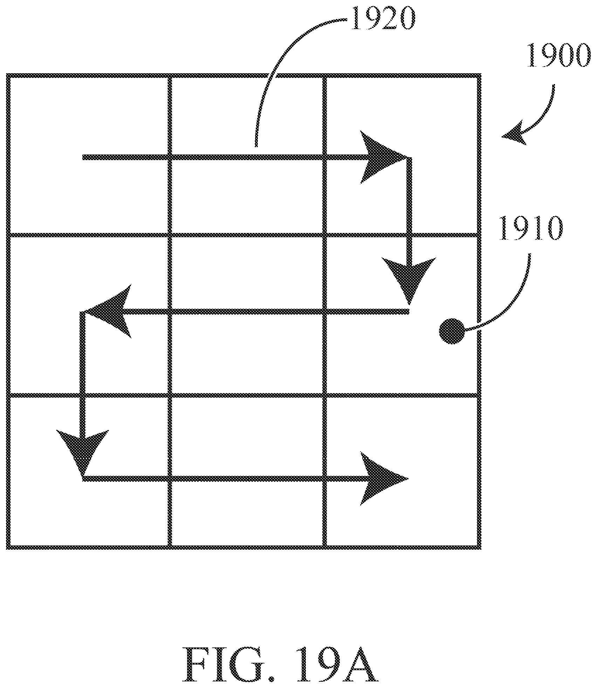

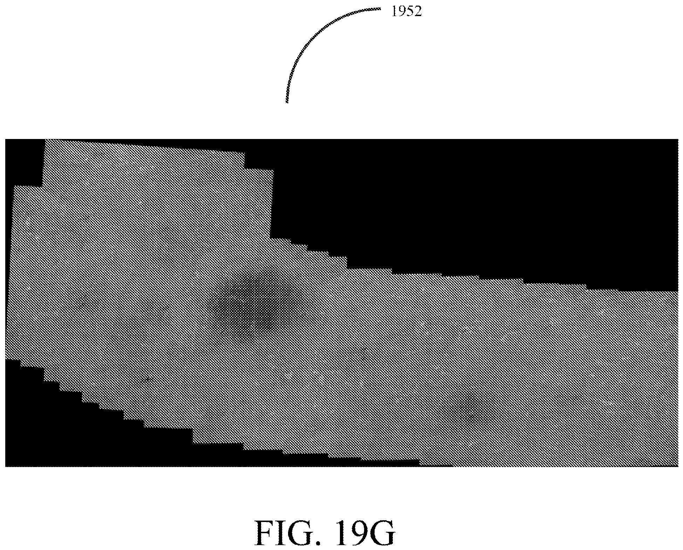

FIG. 19A illustrates an exemplary stitched image (or map) 1900 according to certain exemplary embodiments of the present disclosure. The exemplary stitched image 1900 can comprise a number (e.g., 9) individual images 1910. An exemplary scan path 1920 indicates an exemplary path taken by a view as it traverses a tissue. The illustrated scan path comprises a raster pattern although other patterns are possible (e.g., spiral). Each individual image 1910 can be taken at a point located along the scan path. The stitched image 1900 may be formed from the individual images in several ways. For example, if a position of the view is estimate-able for each individual image (e.g., through scanner feedback), the stitched image 1900 may be constructed through dead-reckoning calculations. Alternatively, the exemplary stitched image 1900 may be constructed using machine vision algorithms for stitching. A first example imaging stitching software is Hugin-Panorama photo stitcher. Hugin is an open source project hosted at http://hugin.Sourceforge.net. A second example image stitching software is a Photomerge tool within Adobe Photoshop. A particular individual embodiment is provided below to further explain tissue imaging in an exemplary EMR treatment device.

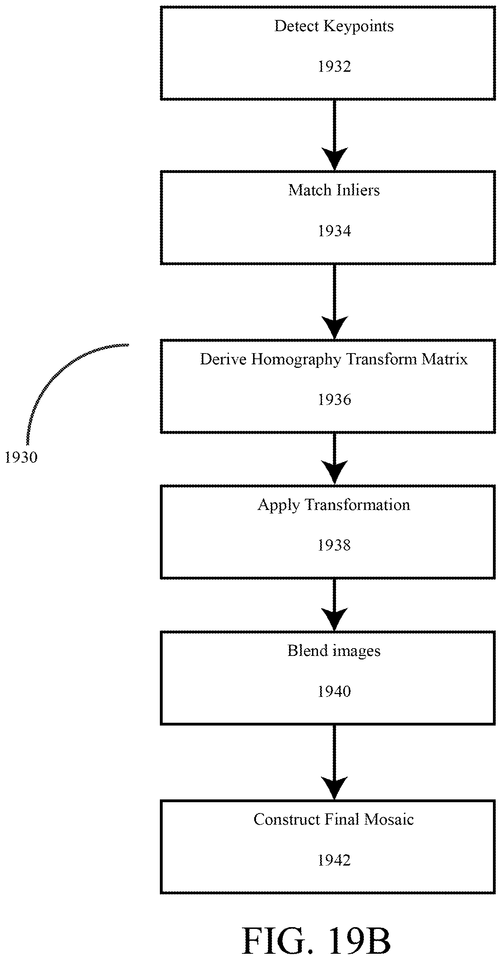

FIG. 19B is a flow diagram that illustrates an exemplary method 1930 for image stitching according to various exemplary embodiments of the present disclosure with which, e.g., a number of images are used to perform the image stitching. First, the method detects keypoints in procedure 1932 within the images. An exemplary keypoint detection method/procedure can be or include scale-invariant feature transform (SIFT). For example, SIFT can apply a Gaussian blur at different scales (e.g., adifferent blur size(s)) to each image, and can determine various exemplary features within each image that have, e.g., the greatest amount of contrast relative adjacent pixels, regardless of the amount of blur.

When a number of keypoints are detected in each image, the keypoints can be compared between overlapping (e.g., sequential) images to match inliers in procedure 1934. An exemplary method of matching keypoints in procedure 1934 can be or include a random sampling consensus (RANSAC). RANSAC is an iterative method/procedure which can be used to estimate parameters of a mathematical model from a set of observed data that contains outliers. For example, RANSAC can iteratively determine inliers, by eliminating outliers from the set of keypoints used to fit the images. When the inliers are determined in procedure 1934, the exemplary method 1930 can derive a homography transform in procedure 1936 to align the images to or with one another based upon the inliers. Homography transform matrices can be derived to relate each image to its overlapping partners. Although homography transforms are given by way of example, it should be understood that other transformation matrices can be used, for example, including but not limited to affine transforms, etc.

The exemplary method 1930 can proceed by applying the transform matrices in procedure 1938, and transforming each image to fit with its adjoining partners (e.g., shifting, scaling, rotation, tilting, tipping, etc.). The exemplary method 1930 can further continue by blending the images in procedure 1940. For example, after the exemplary transformation, a clear juxtaposition can be visible between edges of each image within a final stitched mosaic. In order to prevent such situation, the images can be blended in procedure 1940. An exemplary procedure of image blending 1940 can include, e.g., determining a boundary between adjoining images having a minimal error (e.g., minimal error boundary). The minimal error boundary can be determined by, e.g., analyzing an overlapping portion of two or more images and determining within the overlapping portion on boundary (e.g., non-straight line), where the overlap error is the smallest. For example, an overlap integral can be used to calculate the overlap error. Once the minimum error boundary is determined, the images can be cropped along the minimum error boundary. Further, the exemplary method 1930 can construct the final mosaic in procedure 1942, for example, by digitally positioning all the manipulated images together into a mosaic.

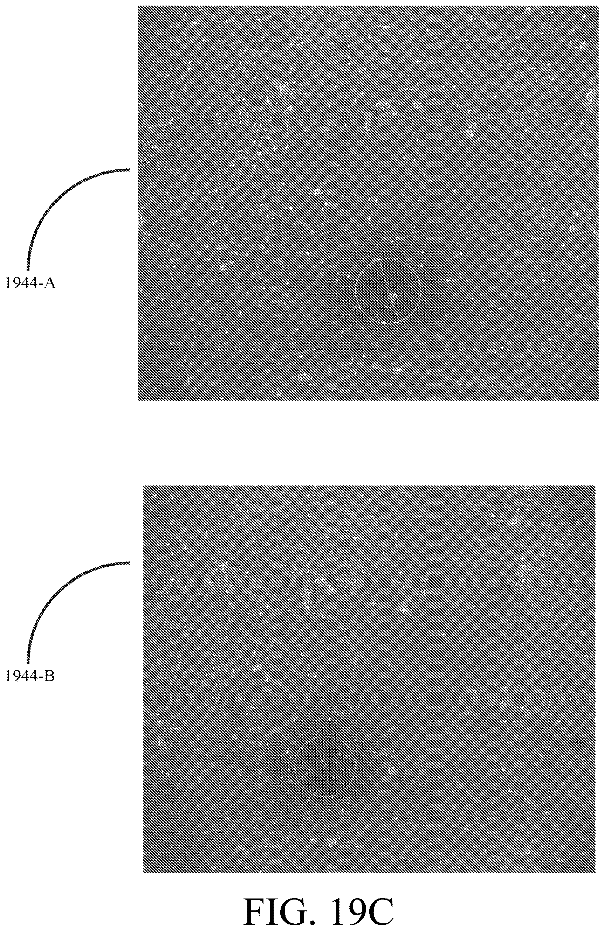

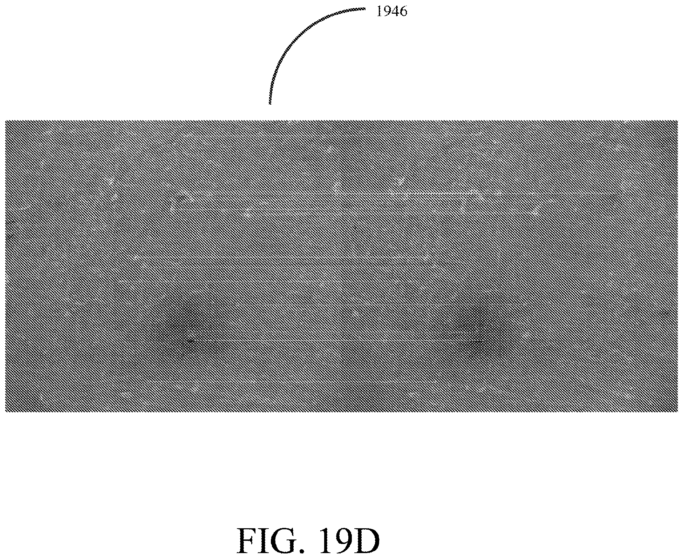

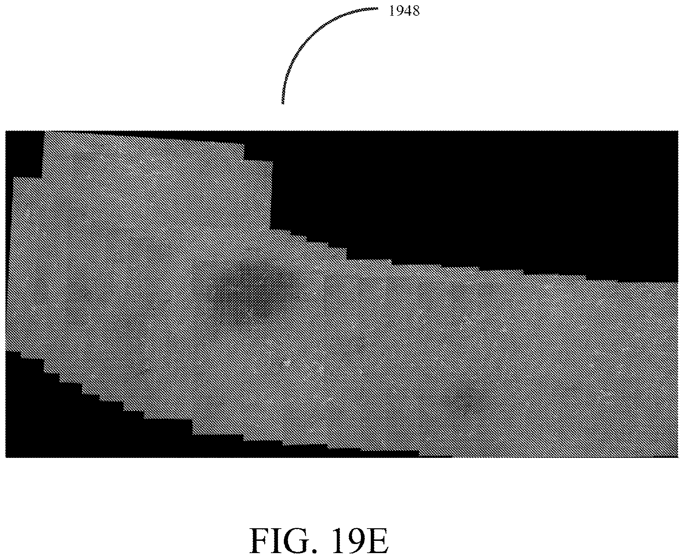

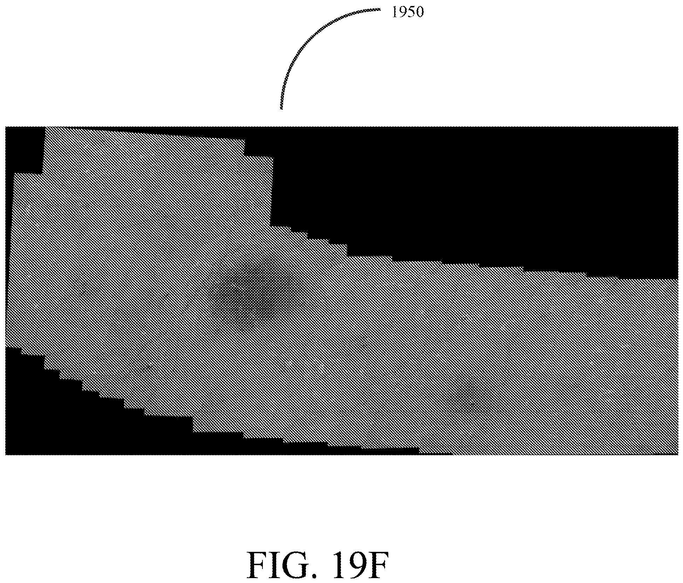

FIG. 19C illustrates two overlapping images 1944-A, 1944-B of skin captured according to certain exemplary embodiments of the present disclosure. As shown in FIG. 19C, the exemplary Keypoint detection of procedure 1932 has been performed on the two images, and detected keypoints are shown therein. FIG. 19D shows the two overlapping images during inlier matching of procedure 1934. The two overlapping images are shown in FIG. 19D as a merged image 1946 with inliers being highlighted. FIG. 19E illustrates an initial unblended mosaic 1948 which comprises the two images 1944-A, 1944-B of FIGs. 19C and 19B. It can be seen that the unblended mosaic comprises hard demarcations of large contrast between overlapping images. FIG. 19F shows a blended mosaic 1950, which is the unblended mosaic 1948 of FIG. 19E after undergoing the blend image procedure 1940. Minimum error boundaries are shown in FIG. 19F in the blended mosaic 1950.

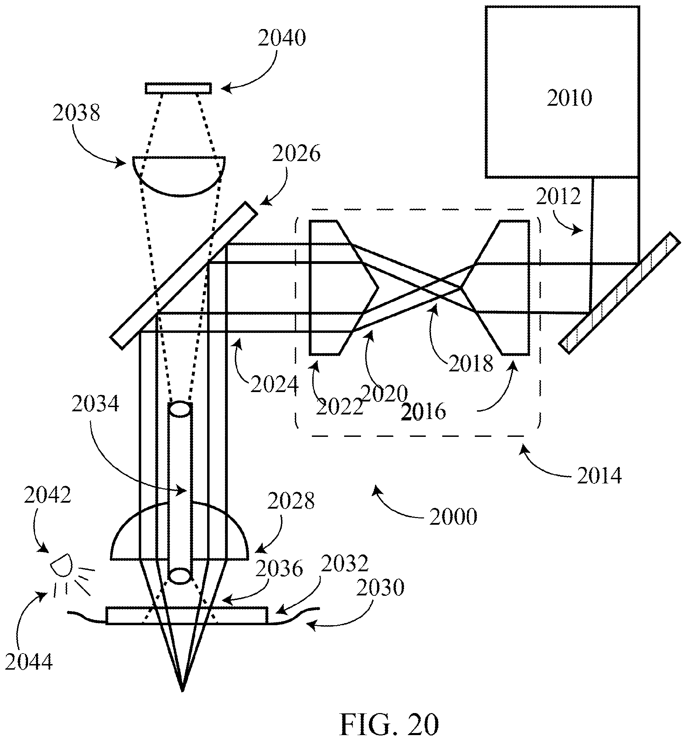

FIG. 20 illustrates an exemplary electromagnetic radiation (EMR) source (e.g., laser source) 2010 generates an EMR beam (e.g., laser beam) 2012 in according to particular exemplary embodiments of the present disclosure. According to certain exemplary embodiments, the EMR beam 2012 can have a transverse ring mode (e.g., TEM 01*) natively from the EMR source 2010. According to other exemplary embodiments, a beam shaper 2014 shapes the EMR beam to produce a transverse ring mode. As shown in FIG. 20, a beam shaper 20114 is provided that employs two axicons. A first axicon 2016 having a first wedge angle can accept the EMR beam 2012 and produce a quasi-Bessel beam. The quasi-Bessel beam can then propagate to produces a diverging ring mode. The diverging ring mode 2020 can be collimated by a second axicon 2022 into an EMR beam having a transverse ring mode 2024. According to certain exemplary embodiments, the ring mode 2024 can be reflected by a beam splitter 20126 and directed toward a focus optic 2028. Some examples of the focus optic 2028 can include converging optics (e.g., plano-convex lenses) and axicons. The focus optic 2028 can converge the EMR beam and direct it toward a tissue 2030 (e.g., skin). According to certain exemplary embodiments, a window 2032 can be located between the focus optic 2028 and the tissue 2030. The window 2032 can be transparent at multiple wavelengths, for example at visible wavelengths and at an EMR wavelength of the EMR beam 2024. Exemplary window materials can include glass, quartz and sapphire. In certain exemplary embodiments, the window 2032 can be cooled and may be used to cool the tissue 2030 during treatment. Commonly, the window 2032 can be placed in contact with an outer surface of the tissue during operation of the exemplary apparatus 2000. The focus optic 2028 can be manufactured with an aperture through its center.

According to certain exemplary embodiments, an optical assembly 2034 can be located within the aperture of the focus optic 2028. The optical assembly 2034 can affect light 2036 from the tissue 2030. In certain exemplary embodiments, the optical assembly 2034 can have an optical axis that is substantially coaxial with an optical axis of the focus optic 2028. According to certain exemplary embodiments, the light 2036 can be transmitted through the beam splitter 2026, and focused by a camera lens 2038 onto a sensor 2040. For example, the sensor 20140 - in some exemplary versions can be or include a camera sensor (e.g., a charge-coupled device [CCD] or Complementary metal-oxide-semiconductor [CMOS] camera). According to certain exemplary embodiments, the tissue 2030 can be illuminated by an illuminator source 2042, which can direct an illuminating light 2044 toward the tissue 2030.

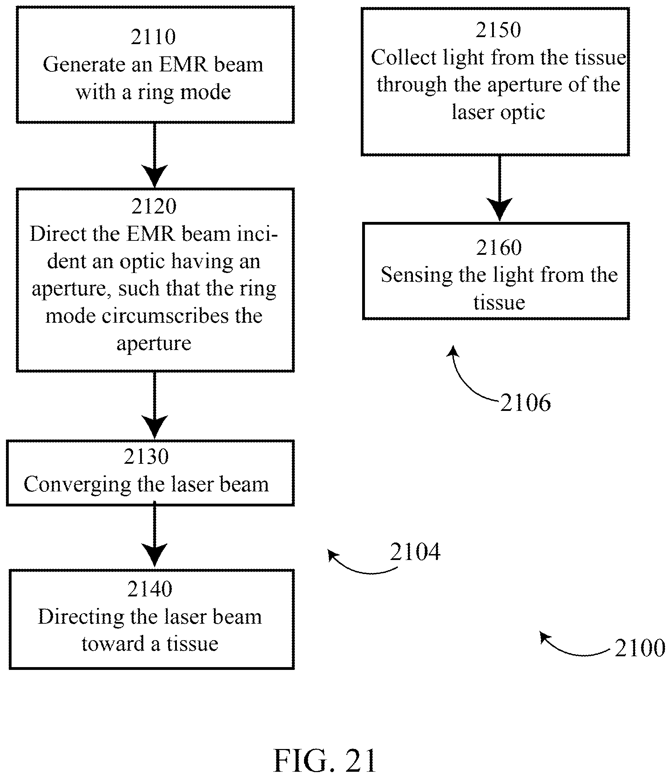

FIG. 21 illustrates a flow diagram for a combined exemplary method 2100 involving treatment and visualization according to certain exemplary embodiments. The exemplary treatment and visualization method 2100 and/or procedures thereof may occur sequentially, coincidently, and/or independent of one another. For this reason, the treatment exemplary method 2104 and the exemplary visualization method 2106 are shown in parallel. Referring initially to the exemplary treatment method 2104, an electromagnetic radiation (EMR) beam having a transverse ring mode can be generated in procedure 2110. An exemplary EMR beam can be a laser beam, and, for example, a 1064nm wavelength laser. An exemplary transverse ring mode can be a transverse electromagnetic mode (TEM) 01* or doughnut mode. Further, the EMR beam can be directed incident an EMR optic having an aperture, such that the transverse ring mode circumscribes the aperture in procedure 2120. In some exemplary versions, the EMR optic can comprises a converging lens and/or an axicon. When the EMR beam has a transverse ring mode, a center portion of the EMR beam can have a negligible radiative power. The EMR beam can be directed to be incident on the EMR optic such that this center portion of the EMR beam can overlap with the aperture of the EMR optic. This way substantially all the radiative power of the EMR beam can be affected by the EMR optic, despite the laser optic having an aperture through its middle portion. The EMR beam can then be converged in procedure 2130, and directed toward a tissue in procedure 2140 by the EMR optic. In certain exemplary embodiments, the converging EMR beam can perform a therapy on the tissue (e.g., photothermolysis). In some additional exemplary embodiments, the exemplary treatment method 2104 can additionally include shaping the EMR beam in order to produce the transverse ring mode, for example, with a beam shaper.

Referring to the exemplary visualization method 2106, light from the tissue is collected through the aperture of the EMR optic 20250. In certain exemplary embodiments, the light from the tissue is directed through the aperture using one or more optical elements. For example, in certain exemplary embodiments, a lens assembly and/or an endoscope is used to collect light through the aperture. In some exemplary versions, the one or more optical elements have an optical axis that is substantially collinear with an optical axis of the EMR optic. According to certain exemplary embodiments, the exemplary combined method 2100 can additionally include separating the light from the tissue from the beam path of the EMR beam, for example, by using a beam splitter. Further, the collected light can be sensed in procedure 2160. According to certain exemplary embodiments, the collected light can be focused to an image, which can then be sensed by a camera sensor (e.g., a charge-coupled device [CCD] or Complementary metal-oxide-semiconductor [CMOS] camera). Then, the camera sensor can produce a digital image of the tissue. This digital image can be used by the operating clinician in order to in alternative embodiments, the light is sensed by alternative ways, for example, a photosensor, a photodiode, and/or a photovoltaic. In some additional exemplary embodiments, the exemplary method can include directing an illumination light toward the tissue, in order to illuminate the tissue for visualization.

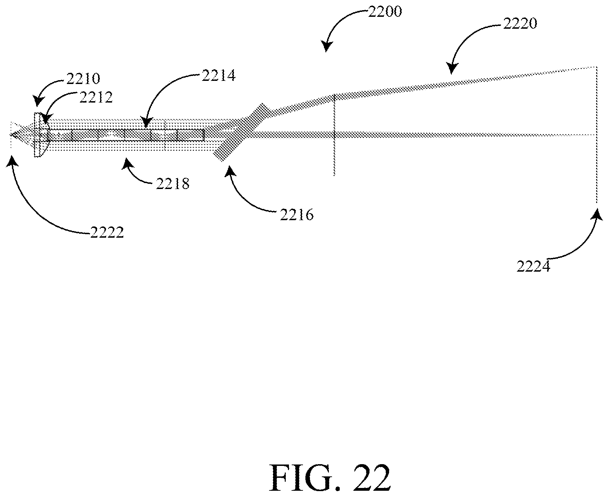

FIG. 22 shows a diagram of a ray-trace 2200, using the exemplary system(s) and/or method(s) according to certain exemplary embodiments of the present disclosure. For example, as illustrated in FIG. 22, a focus optic 2210 can have an aperture 2212 through a center thereof. An endoscope 2214 can be provided through the aperture 2212. A beam splitter 2216 can be placed following the endoscope 2214 in the beam path. The beam splitter 2216 can be configured to reflect a laser beam wavelength (e.g., 1064nm) and pass light wavelengths for sensing (e.g., visible wavelengths). Such exemplary paths of the exemplary rays 2218, 2220 are shown in FIG. 22. The exemplary laser ray trace 2218 illustrates a path of rays associated with a treatment laser. The exemplary imaging ray trace 2220 illustrates a path of rays associated with the endoscope 2216. An exemplary object plane 2222 and an exemplary image plane 2224 are shown in FIG. 2.

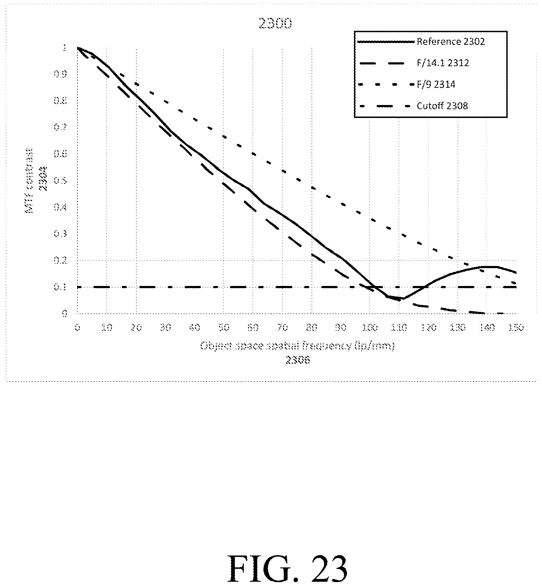

FIG. 23 illustrates a modulation transfer function (MTF) graph 2300 for a diffraction limited endoscope imaging systems according to an exemplary embodiment of the present disclosure, compared with a DermLite Foto II Pro photographic dermatoscope lens assembly 2302. The DermLite Foto II Pro is currently available to the market from 3Gen, Inc. of San Juan Capistrano, California, U.S.A. The graph 20400 depicts MTF contrast on a vertical axis 20404 and spatial frequency along a horizontal axis 20406. A cutoff frequency 20408 has been arbitrarily selected to be at an MTF contrast value of 10%. A F/14.1 diffraction limited endoscope 20412 and a F/9 diffraction limited endoscope 20414 have best case MTF curves plotted on the graph 20400. As the endoscope MTF curves in the graph 20400 are diffraction limited, and therefore the performance of an actual endoscope system will be less than that shown in the graph. For this reason, a test was performed in order to quantify actual performance achievable with an exemplary endoscope-based imaging system.

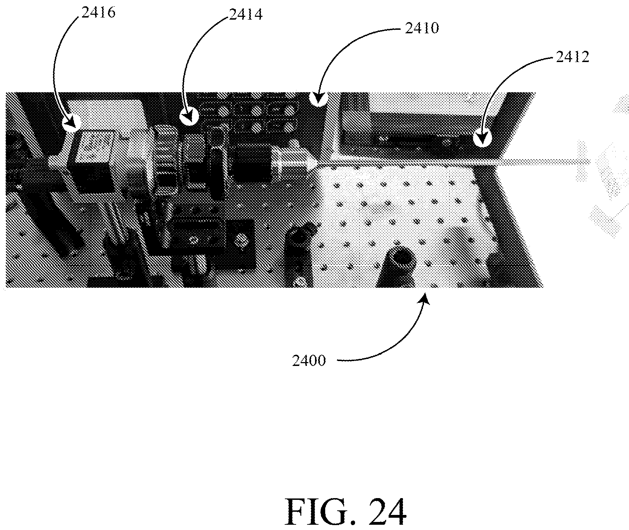

FIG. 24 shows an exemplary image 2400 of an exemplary configuration 2410 for an exemplary endoscope imaging system according to an exemplary embodiment of the present disclosure. The exemplary system/configuration 2410 comprises an endoscope 2412, a coupling lens 2414, and a camera 2416. The endoscope can be, e.g., a Hawkeye ProSlim from Gradient Lens Corporation of Rochester, New York, U.S.A. The Hawkeye ProSlim used in the tests had a length of 7", an outside diameter of 4.2mm, a field of view (FOV) of 42°, and a small illuminated ring light. A coupler optical assembly 2414 can be attached to the endoscope 2412. Examples of coupler optical assemblies can include: 18mm, 20mm, and 30mm focal length assemblies. Finally, the coupler optical assembly 2414 can be attached to a camera 2416. An example of a camera can include a Basler ACA2500-14UC from Basler of Ahrensburg, Germany.

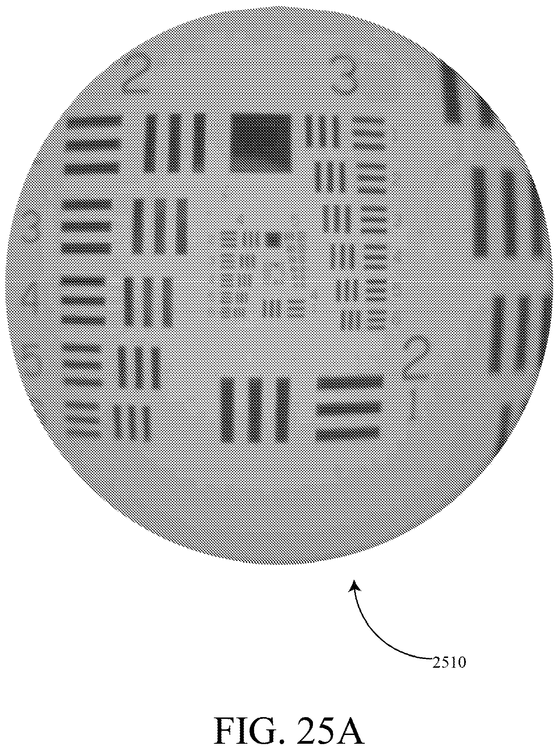

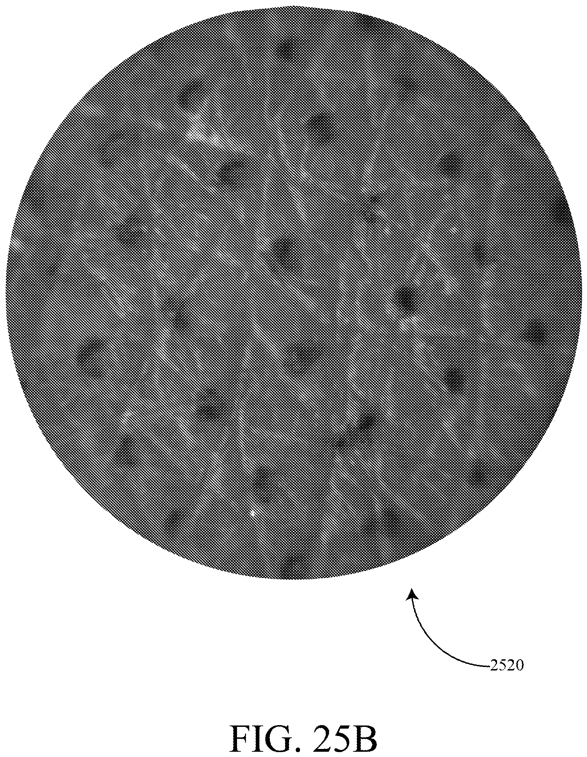

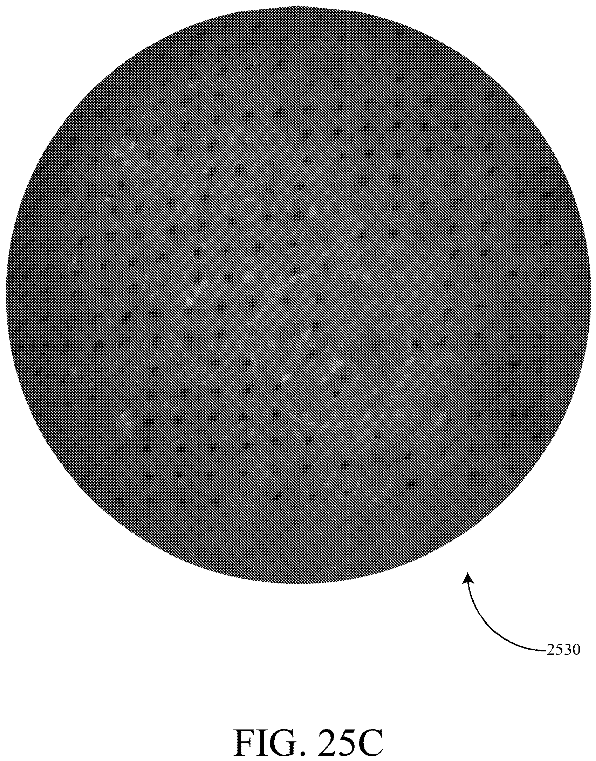

FIGS. 25A-25C illustrate exemplary images from the exemplary configuration 2510. A first exemplary image 2510 is shown in FIG. 25A, and was taken with a 30mm focal length coupler lens and the Basler ACA2500-14UC camera. The first exemplary image 2510 illustrates a 1952 Air Force target taken at focus. A second exemplary image 2520 is shown in FIG. 25B, and was taken with a 20mm focal length coupler and a PixeLink PL-D755 camera from PixeLink of Ottawa, Ontario, Canada. The second exemplary image 2520 illustrates a skin region treated with a fractionated pattern at a first magnification. A third exemplary image 2530 is shown in FIG. 25C, and was taken with a 20mm focal length coupler and a PixeLink PL-D755 camera from PixeLink of Ottawa, Ontario, Canada. The third exemplary image 2530 illustrates a skin region treated with a fractionated pattern at a second magnification.

### Additional Exemplary Embodiments

Additional exemplary embodiments include alternative imaging technologies used in conjunction with EMR-based treatment. These alternative imaging technologies can include: microscopic imaging, wide field of view imaging, reflectance confocal imaging, optical coherence tomography imaging, optical coherence elastography imaging, coherent anti-stokes Raman spectroscopy imaging, two-photon imaging, second harmonic generation imaging, phase conjugate imaging, photoacoustic imaging, infrared spectral imaging, and hyperspectral imaging.

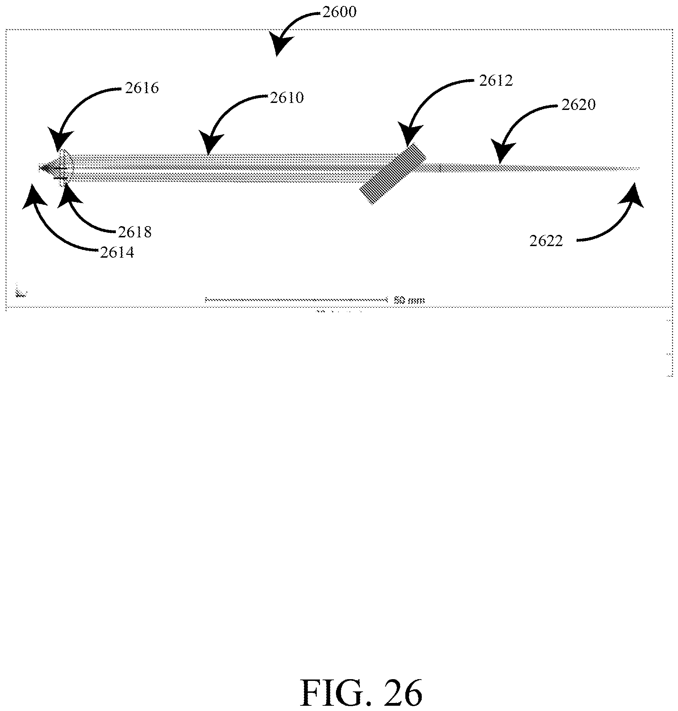

A diagram of an exemplary ray trace 2600 using the exemplary system(s) and/or method(s) according to an additional exemplary embodiment of the present disclosure is shown in FIG. 26. For example, annular laser beam rays 2610 are shown there as being reflected from a beam splitter 2612. The laser beam rays 2610 are then focused to a tissue plane 2614 by an aspherical focus optic 2616. The focus optic 2616 can have a hole 2618 through its center. The image rays 2620 pass through the hole 2618, and extend from a point source at the tissue plane 2614. The image rays 2620 are transmitted through the beam splitter 2612. Following the beam splitter 2612 in the beam path, an extra long working distance microscope objective can be provided that can bring the image rays to focus at an image plane 2622. Such exemplary extra-long working distance microscope objective can be, e.g., InfiniMini from Photo-Optical Company of Boulder, Colorado, U.S.A. In certain exemplary embodiments of the present disclosure, the exemplary extra-long working distance microscope objective can be coupled to a standard converter and an LDS amplifier (e.g., both also can be from Photo-Optical Company) to provide a 2.4mm field of view (FOV), a 110mm working distance (WD), and 106 line pair per mm (lpmm) resolution with an f-number of about f-14. According to yet another exemplary embodiment of the present disclosure, the image rays 2620 still pass through a central aperture 2618 of the focus optic 2616, but without the use of an exemplary optical arrangement (e.g., endoscope) located within the aperture 2618. Instead, the extra-long working distance objective can obviate the need for imaging optics on the object side of the beam splitter 2612.

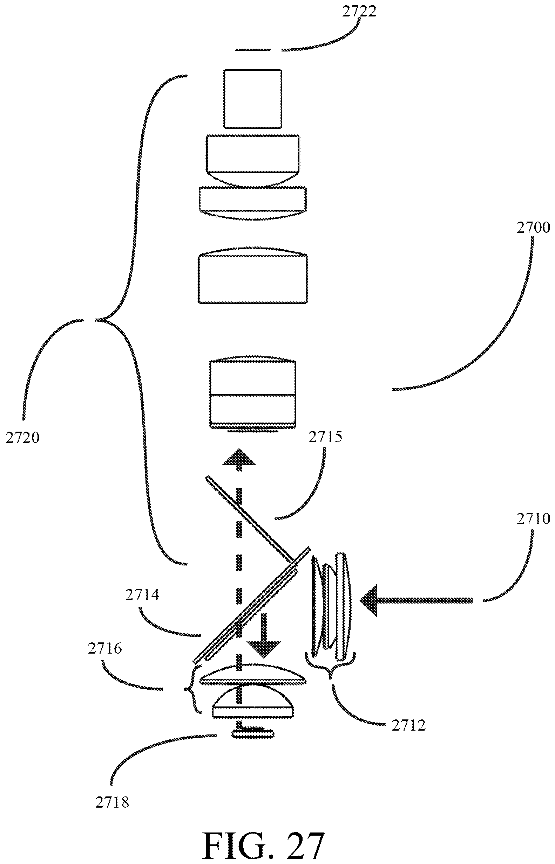

FIG. 27 shows another exemplary embodiment of a data collection and treatment device/system 2700 according to the present disclosure, and the exemplary operation thereof. As provided in FIG. 27, the exemplary device/system 2700 can direct and focus a therapeutic electromagnetic radiation (EMR) beam 2710. Exemplary EMR beams can include, e.g., high quality lasers (e.g., M²<1.5). For example, in some exemplary cases, the EMR beam 2710 can utilize a wavelength in a range between about 800nm and about 1200nm, a pulse energy in a range between about 10 mJ and about 10,000 mJ, and a pulse duration in a range between about 5nsec and about 150nsec. The EMR beam 2710 can be first acted upon a first lens optic group 2712. In some exemplary embodiments, the first optic group 2712 can comprise a diffractive optical element (DOE) to split the laser beam into a plurality of beamlets of different angular tilts/tips that focus into a 2D patterned array. Examples of DOEs and their use in similar applications are described in, e.g., U.S. Pat. Appl. No. 16/381,736, the entirety of which is incorporated herein by reference. An exemplary DOE can be Holo/OR Part No. MS-429-I-Y-A, which produces an 5X5 array of beamlets, from Holo/OR of Ness Ziona, Israel.

After passing the first optic group 2712, the EMR beam 2710 can be reflected by a beam splitter 2714. The beam splitter - in some exemplary cases - can be configured to reflect the EMR beam 2710, and transmit light 2715. Exemplary beam splitters can include, e.g., notch, low-pass, and/or high-pass filters. After being reflected by the beamsplitter 2714, the EMR beam 2710 can pass through a second optic group 2716. The second optic group 2716 and the first optic group 2712 are designed and/or configured to work in concert to focus the EMR beam (or plurality of EMR beamlets) 2710 to a focal region that is located down stream, e.g., at a prescribed distance away (e.g., between about 0 -1.5mm +/- 0.02mm), from a contacting window 2718, for example, within a tissue. In some exemplary embodiments, the first optic group 2712 and the second optic group 2716 can together comprise a folder Petzval lens.

The light 2718, for example, from a surface of the tissue can be directed back up through the contacting widow 2718, the second optic group 2716, the beam splitter 2714 and imaged by a third optic group 2720. The third optic group 2720 and the second optic group 2716 can act in concert to reimage a return light 2715 to and/or on a sensor plane 2722, where a camera sensor (e.g., CMOS or CCD sensor) can be located. The camera sensor can be configured to capture digital data (e.g., images) representative of the reimaged light 2715. In some exemplary embodiments, the light 2715 originating from the tissue placed in contact with an outer face of the contacting window 2718 can be brought into focus at a sensor plane 2722. In this exemplary case, the light 2715 can typically have a wavelength, e.g., in the visible range, as this range of radiations being less transmissive (therefore less penetrative) in the tissue. Alternatively or in addition, the light 2715 can originate from a position at a known distance away (e.g., between about 0 -1.5mm +/- 0.02mm) from the window 2718 that is brought into focus at the sensor plane 2722. In this exemplary alternative/additional case, the light 2715 can typically be selected having a wavelength in the near-infrared range, e.g., because in this range of wavelengths tissue is more transmissive.

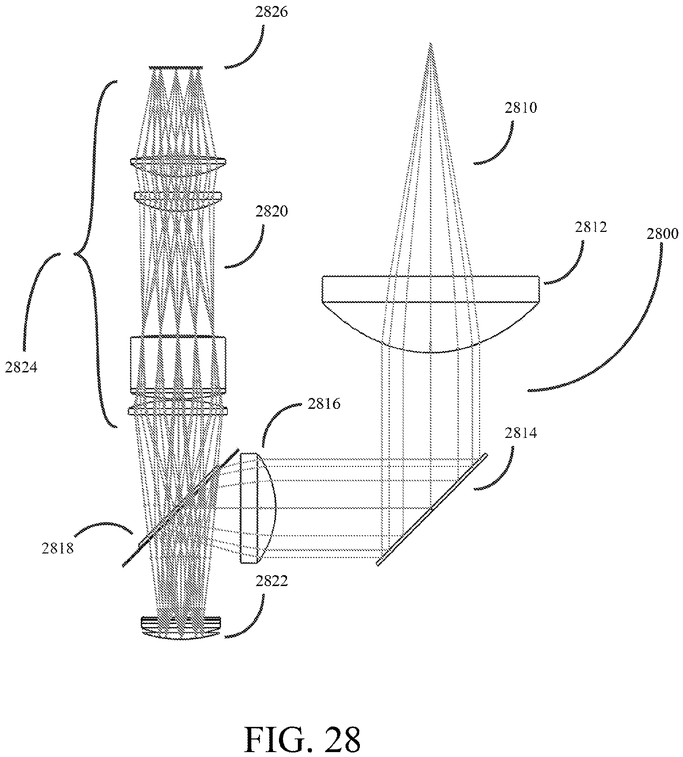

FIG. 28illustrates another exemplary data collection and treatment system 2800 according to yet further exemplary embodiment of the present disclosure. As shown in FIG. 28, the system 2800 can be configured to direct and focus an electromagnetic radiation (EMR) beam 2810 toward a focal region. The EMR beam 2810 is first shown in FIG. 28 as being diverging, and then it is collimated by a collimation optic 2812. The curvature of the collimation optic 2812 can be selected to based upon a rate of divergence of the EMR beam 2810. The collimated EMR beam can then be reflected by a mirror 2814 to be incident on and to a focus optic 2816. Another focus optic 2816 can converge the EMR beam 2810 at a high rate (e.g., NA greater than about 0.2). The converging EMR beam 2810 can then be selectively reflected by another beamsplitter 2818 which can be configured to reflect the EMR beam 2810 and transmit light 2820 for a subsequent detection. In some exemplary embodiments, the light 2820 for detection is within a visible range (e.g., about 350-750nm) and the EMR beam 2810 can be outside of the visible range.

The EMR beam 2810 can then be finally directed through a window 2822, which is configured to be placed in contact with a tissue during treatment. The EMR beam 2810 - in various exemplary embodiments - can be configured to be focused at a focal region that is located downstream (e.g., outside of) at a prescribed distance (e.g., between about 0 -1.5mm +/- 0.02mm) from the window 2822. The light 2820 originated from the tissue can be transmitted through the window 2822, and then imaged by an optical assembly 2824 that brings the light to focus on or at a sensor plane 2826. A camera sensor can be placed at the sensor plane 2826, and used to captured digital data associated with or representative of the light 2820. In some exemplary embodiments, the light 2820 originating from tissue placed in contact with an outer face of the window 2822 can be brought into focus at the sensor plane 2826 by the optical assembly 2824. In this exemplary case, the light 2820 can have a wavelength in the visible range, as having the wavelength within such exemplary range tissue that is less transmissive. Alternatively or in addition, the light that originates from a position at a known distance away (e.g., between about 0 -1.5mm +/- 0.02mm) from the window 2822 can be brought into focus at the sensor plane 2826 by the optical assembly 2824. In this exemplary alternative or additional case, the light 2820 can be selected as having a wavelength in the near-infrared range, because in this range of wavelengths, the tissue is more transmissive.
In the following, specific examples are listed.
1. An apparatus for treating at least one patient, comprising:
   a data collection System configured to collect data for the at least one patient;
   a Controller configured to:
      authenticate access to a remote network,
      aggregate the collected patient data, and
      cause a storage of the aggregated patient data on a data storage device which is in communication with the remote network;
      an electromagnetic radiation ("EMR") source configured to generate an EMR beam;
      an optics arrangement configured to converge or focus the EMR beam to a focal region located (i) along an optical axis within at least one portion of the at least one patient,
      and (ii) below a surface of a tissue of the at least one patient; and
      a window located at a predetermined distance away from the focal region, and
      provided between the focal region and the optics arrangement along the optical axis, wherein
      the window is configured to transmit the EMR beam, and contact the surface of the tissue of the at least one patient.
2. The apparatus of example 1, wherein the Controller is further configured to access a module which is in communication with the remote network, and wherein the module comprises at least one of an image recognition module, a Computer vision module, an electronic health record module, or a clinical decision-making Support module.
3. The apparatus of example 1, wherein the data of the at least one patient comprises at least one of an image of patient tissue, an age of patient, treatment session Information, a patient pain score, a data collection parameter, or an EMR-based treatment parameter.
4. The apparatus of example 1, wherein the data collection System is configured to collect the patient data from the tissue which is in contact with the window, and wherein the data collection System and the optics arrangement are spatially registered to the window.
5. The apparatus of example 1, further comprising a drug-based treatment System configured to be utilized in a drug-based treatment of the at least one patient.
6. The apparatus of example 5, wherein the drug-based treatment System comprises at least one of a topical drug, an injectable drug, or an orally-delivered drug.
7. The apparatus of example 1, wherein the optics arrangement is configured to converge or focus the laser beam at a numerical aperture (NA) of at least 0.3.
8. The apparatus of example 1, wherein the data collection System comprises:
   an Illumination source configured to illuminate the surface of the tissue;
   a light-directing arrangement configured to direct light from the surface of the tissue through the window to a sensor plane; and
   a sensor arrangement configured to detect the light at the sensor plane, wherein the collected patient data comprises a plurality of images.
9. The apparatus of example 8, wherein the Controller is configured to aggregate the collected patient data by stitching together the plurality of images.
10. The apparatus of example 1, wherein the data collection System comprises at least one of (i) a user interface configured to accept the data of the at least one patient from a user, or (ii) a System interface configured to accept the data of the at least one patient from a further network connected to a storage device containing the data of the at least one patient.
11. The apparatus of example 1, wherein the data collection System comprises at least one of photoacoustic imaging System, a camera, a dermatoscope Subsystem, a microscope Subsystem, a confocal microscope Subsystem, a plasma detection Subsystem, or a window referencing Subsystem.
12. The apparatus of example 1, wherein the Controller is further configured to access a module which is in communication with the remote network by performing an authentication with the module.
13. The apparatus of example 12, wherein the authentication is performed by verifying that at least one of (i) a financial agreement is in place, (ii) a financial distribution has been received, or (iii) the financial distribution is pending.
14. The apparatus of Example 12, wherein the authentication is performed by effectuating a financial distribution of a fee.
15. The apparatus of example 14, wherein the fee is provided for at least one of a treatment, a patient, a subscription, an image, or a Service module.
16. The apparatus of example 1, wherein the optics arrangement comprises a folded Petzval lens.
17. A method for treating at least one patient, comprising:
   with a data collection System, collecting data for the at least one patient;
   aggregating the collected patient data;
   authenticating access to a remote network;
   storing the patient data to a data storage device in communication with the remote network;
   with an electromagnetic ("EMR") source, generating an EMR beam;
   with an optics arrangement, converging or focusing of the EMR beam to a focal region located (i) along an optical axis, and (ii) below a surface of a tissue of the at least one patient;
   contacting the surface of the tissue of the at least one patient with a window that is located at a predetermined distance away from the focal region, and between the focal region and the focus optic along the optical axis; and
   transmitting the EMR beam through the window, wherein the focal region is positioned within the tissue.
18. The method of example 17, further comprising accessing a module which is in communication with the remote network, wherein the module comprises at least one of an image recognition module, a Computer vision module, an electronic health record module, or a clinical decision-making Support module.
19. The method of example 17, wherein the data of the at least one patient comprises at least one of an image of patient tissue, an age of patient, treatment session Information, a patient pain score, a data collection parameter, or an EMR-based treatment parameter.
20. The method of example 17, wherein collecting the patient data comprises sensing the patient data firom the tissue which is in contact with the window, and wherein the data collection System and the optics arrangement are spatially registered to the window.
21. The method of example 17, further comprising performing a drug-based treatment on the at least one patient.
22. The method of example 21, wherein the drug-based treatment comprises at least one of a topical drug, an injectable drug, or an orally-delivered drug.
23. The method of example 17, wherein converging or focusing the electromagnetic radiation (EMR) beam to the focal region is performed at a numerical aperture (NA) of at least 0.3.
24. The method of example 17, wherein collecting patient data additionally comprises:
   illuminating the surface of the tissue of the at least one patient;
   directing light from the surface of the tissue through the window to an image plane; and
   sensing the light at the image plane using a sensor arrangement, wherein the collected patient data comprises a plurality of images.
25. The method of example 24, wherein aggregating the collected patient data comprises stitching together the plurality of images.
26. The method of example 17, wherein the collecting of the data further comprises at least one of (i) inputting patient data using a user interface, or (ii) interfacing with a further network facilitating a storage device that contains the data of the at least one patient.
27. The method of Example 17, wherein the collecting of the data comprises using at least one of an photoacoustic imaging apparatus, a camera, a dermatoscope Subsystem, a microscope Subsystem, a confocal microscope Subsystem, a plasma detection Subsystem, or a
   window referencing Subsystem.
28. The method of Example 17, further comprising accessing a module in communication with the remote network by authenticating access to the module.
29. The method of Example 27, wherein the authentication is performed by verifying that at least one of (i) a financial agreement is in place, (ii) a financial distribution has been received, or (iii) the financial distribution is pending.
30. The method of Example 27, wherein the authentication is performed by effectuating a financial distribution of a fee.
31. The method of example 29, wherein the fee is provided for at least one of a treatment, a patient, a subscription, an image, or a Service module.
33. The method of example 17, wherein the optics arrangement comprises a folded Petzval lens.
34. A computer-accessible medium having Computer Software thereon for facilitating a treatment of at least one patient, wherein, when the Computer Software is executed by a Computer processor, the Computer processor is configured to perform procedures comprising:
   with a data collection System, collecting data for the at least one patient;
   causing an aggregation of the collected patient data;
   causing an authentication of access to a remote network;
   storing the patient data to a data storage device in communication with the remote network;
   Controlling an electromagnetic radiation ("EMR") source to generate an EMR beam;
   Controlling an optics arrangement to converge or focus the EMR beam to a focal region located (i) along an optical axis, and (ii) below a surface of a tissue of the at least one patient;
   Controlling contacting of the surface of the tissue of the at least one patient with a 5 window that is located at a predetermined distance away from the focal region, and between the focal region and the focus optic along the optical axis; and
   Controlling a transmission of the EMR beam through the window, wherein the data collection System and the optics arrangement are registered to the window, and wherein the focal region is positioned within the tissue.

One skilled in the art will appreciate further features and advantages of the disclosure based on the above-described embodiments. Accordingly, the present disclosure is not to be limited by what has been particularly shown and described, except as indicated by the appended Claims. All publications and references cited herein are expressly incorporated herein by reference in their entireties.

The subject matter described herein can be implemented in digital electronic circuitry, or in Computer Software, firmware, or hardware, including the structural means disclosed in this specification and structural equivalents thereof, or in combinations of them. The subject matter described herein can be implemented as one or more Computer program products, such as one or more Computer programs tangibly embodied in an Information carrier (e.g., in a machine readable storage device), or embodied in a propagated signal, for execution by, or to control the Operation of, data processing apparatus (e.g., a programmable processor, a Computer, or multiple Computers). A Computer program (e.g., also known as a program, Software, Software application, or code) can be written in any form of programming language, including compiled or interpreted languages, and it can be deployed in any form, including as a stand-alone program or as a module, component, subroutine, or other unit suitable for use in a computing environment. A Computer program does not necessarily correspond to a file. A Computer program can be stored or recorded in a portion of a file that holds other programs or data, in a single file dedicated to the program in question, or in multiple coordinated flies (e.g., flies that store one or more modules, sub programs, or portions of code). A Computer program can be deployed to be executed on one computer or on multiple computers at one site or distributed across multiple sites and interconnected by a communication network.

The exemplary processes, method, procedure and logic flows described in this specification, including the method steps of the subject matter described herein, can be performed by one or more programmable processors executing one or more computer programs to perform functions of the subject matter described herein by operating on input data and generating output. The processes and logic flows can also be performed by, and exemplary apparatus of the subject matter described herein can be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processor of any kind of digital computer. Generally, a processor will receive instructions and data from a read only memory or a random access memory or both. The essential elements of a computer are a processor for executing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. Information carriers suitable for embodying computer program instructions and data include all forms of nonvolatile memory, including by way of example semiconductor memory devices, (e.g., EPROM, EEPROM, and flash memory devices); magnetic disks, (e.g., internal hard disks or removable disks); magneto optical disks; and optical disks (e.g., CD and DVD disks). The processor and the memory can be supplemented by, or incorporated in, special purpose logic circuitry.

To provide for interaction with a user, the subject matter described herein can be implemented on a computer having a display device, e.g., a CRT (cathode ray tube) or LCD (liquid crystal display) monitor, for displaying information to the user and a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The exemplary techniques described herein can be implemented using one or more modules. As used herein, the term "module" refers to computing software, firmware, hardware, and/or various combinations thereof. At a minimum, however, modules are not to be interpreted as software that is not implemented on hardware, firmware, or recorded on a non-transitory processor readable recordable storage medium (i.e., modules are not software per se). Indeed "module" is to be interpreted to always include at least some physical, non-transitory hardware such as a part of a processor or computer. Two different modules can share the same physical hardware (e.g., two different modules can use the same processor and network interface). The modules described herein can be combined, integrated, separated, and/or duplicated to support various applications. Also, a function described herein as being performed at a particular module can be performed at one or more other modules and/or by one or more other devices instead of or in addition to the function performed at the particular module. Further, the modules can be implemented across multiple devices and/or other components local or remote to one another. Additionally, the modules can be moved from one device and added to another device, and/or can be included in both devices.

The subject matter described herein can be implemented in a computing system that includes a back end component (e.g., a data server), a middleware component (e.g., an application server), or a front end component (e.g., a client computer having a graphical user interface or a web browser through which a user can interact with an implementation of the subject matter described herein), or any combination of such back end, middleware, and front end components. The components of the system can be interconnected by any form or medium of digital data communication, e.g., a communication network. Examples of communication networks include a local area network ("LAN") and a wide area network ("WAN"), e.g., the Internet.

Approximating language, as used herein throughout the specification and paragraphs, may be applied to modify any quantitative representation that could permissibly vary without resulting in a change in the basic function to which it is related. "Approximately," "substantially,"
or "about" can include numbers that fall within a range of 1%, or in certain exemplary embodiments within a range of 5% of a number, or in certain exemplary embodiments within a range of 10% of a number in either direction (greater than or less than the number) unless otherwise stated or otherwise evident from the context (except where such number would impermissibly exceed 100% of a possible value). Accordingly, a value modified by a term or terms, such as "about," "approximately," or "substantially," are not to be limited to the precise value specified. In at least some instances, the approximating language may correspond to the precision of an instrument for measuring the value. Here and throughout the specification and paragraphs, range limitations may be combined and/or interchanged, such ranges are identified and include all the sub-ranges contained therein unless context or language indicates otherwise.

The articles "a" and "an" as used herein in the specification and in the paragraphs, unless clearly indicated to the contrary, should be understood to include the plural referents. Paragraphs or descriptions that include "or" between one or more members of a group are considered satisfied if one, more than one, or all of the group members are present in, employed in, or otherwise relevant to a given product or process unless indicated to the contrary or otherwise evident from the context. The disclosure includes embodiments in which exactly one member of the group is present in, employed in, or otherwise relevant to a given product or process. The disclosure also includes embodiments in which more than one, or all of the group members are present in, employed in, or otherwise relevant to a given product or process. Furthermore, it is to be understood that the disclosed embodiments provide all variations, combinations, and permutations in which one or more limitations, elements, clauses, descriptive terms, etc., from one or more of the listed paragraphs is introduced into another claim dependent on the same base claim (or, as relevant, any other claim) unless otherwise indicated or unless it would be evident to one of ordinary skill in the art that a contradiction or inconsistency would arise. It is contemplated that all embodiments described herein are applicable to all different aspects of the disclosed embodiments where appropriate. It is also contemplated that any of the embodiments or aspects can be freely combined with one or more other such embodiments or aspects whenever appropriate. Where elements are presented as lists, e.g., in Markush group or similar format, it is to be understood that each subgroup of the elements is also disclosed, and any element(s) can be removed from the group. It should be understood that, in general, where the disclosed embodiments, or aspects of the disclosed embodiments, is/are referred to as comprising particular elements, features, etc., certain embodiments of the disclosure or aspects of the disclosure consist, or consist essentially of, such elements, features, etc. For purposes of simplicity those embodiments have not in every case been specifically set forth in so many words herein. It should also be understood that any embodiment or aspect of the disclosure can be explicitly excluded from the paragraphs, regardless of whether the specific exclusion is recited in the specification. For example, any one or more active agents, additives, ingredients, optional agents, types of organism, disorders, subjects, or combinations thereof, can be excluded.

Where ranges are given herein, embodiments of the disclosure include embodiments in which the endpoints are included, embodiments in which both endpoints are excluded, and embodiments in which one endpoint is included and the other is excluded. It should be assumed that both endpoints are included unless indicated otherwise. Furthermore, it is to be understood that unless otherwise indicated or otherwise evident from the context and understanding of one of ordinary skill in the art, values that are expressed as ranges can assume any specific value or subrange within the stated ranges in different embodiments of the disclosure, to the tenth of the unit of the lower limit of the range, unless the context clearly dictates otherwise. It is also understood that where a series of numerical values is stated herein, the disclosure includes embodiments that relate analogously to any intervening value or range defined by any two values in the series, and that the lowest value may be taken as a minimum and the greatest value may be taken as a maximum. Numerical values, as used herein, include values expressed as percentages.

It should be understood that, unless clearly indicated to the contrary, in any methods claimed herein that include more than one act, the order of the acts of the method is not necessarily limited to the order in which the acts of the method are recited, but the disclosure includes embodiments in which the order is so limited. It should also be understood that unless otherwise indicated or evident from the context, any product or composition described herein may be considered "isolated".

As used herein the term "comprising" or "comprises" is used in reference to compositions, methods, and respective component(s) thereof, that are essential to the disclosed embodiments, yet open to the inclusion of unspecified elements, whether essential or not.

As used herein the term "consisting essentially of" refers to those elements required for a given embodiment. The term permits the presence of additional elements that do not materially affect the basic and novel or functional characteristic(s) of that embodiment of the disclosure.

The term "consisting of" refers to compositions, methods, and respective components thereof as described herein, which are exclusive of any element not recited in that description of the embodiment.

Although a few variations have been described in detail above, other modifications or additions are possible.

In the descriptions above and in the paragraphs, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," above and in the paragraphs is intended to mean, "based at least in part on," such that an unrecited feature or element is also permissible.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and sub-combinations of the disclosed features and/or combinations and sub-combinations of several further features disclosed above. In addition, the logic flows depicted in the accompanying figures and/or described herein do not necessarily require the particular order shown, or sequential order, to achieve desirable results. Other implementations may be within the scope of the following claims.

## Claims

1. A system, comprising:
an electromagnetic radiation (EMR) treatment device configured to generate a treatment EMR beam to treat a patient tissue afflicted with a condition, the EMR treatment device being operable at a set of base parameters;
at least one processor; and
at least one non-transitory computer-readable storage medium storing instructions thereon that, when executed by the at least one processor, cause the at least one processor to:
receive patient data from a remote network in electronic communication with the at least one processor, the patient data including at least one pretreatment image of the patient tissue;
determine one or more recommended changes to the set of base treatment parameters based on the received patient data;
offer the determined one or more recommended changes to a treatment operator; and
append the patient data with at least one posttreatment image of the patient tissue.

2. The system of claim 1, wherein the one or more recommended changes can include adjustments to laser pulse energy of the EMR beam, focus depth of the EMR beam, and energy density over the patient tissue.

3. The system of claim 1, wherein the one or more recommended changes are determined using a machine-learning algorithm.

4. The system of claim 1, wherein the instructions further cause the at least one processor to compare the at least one pretreatment image of the patient tissue and the at least one post treatment image of the patient tissue to grade progression of a treatment of the patient tissue with the EMR treatment device.

5. The system of claim 1, wherein the instructions further cause the at least one processor to determine, from color analysis, a skin type of the patient tissue.

6. The system of claim 1, wherein the instructions further cause the at least one processor to organize and provide at least some of a stored patient data portfolio to create an individualized treatment plan.

7. The system of claim 1, wherein the instructions further cause the at least one processor to authenticate access to the remote network prior to receiving the patient data therefrom.

8. The system of claim 7, wherein authentication is based at least partially on a verification of a current payment status.

9. A method, comprising:
receiving, at a treatment system including an electromagnetic radiation (EMR) treatment device configured to treat patient tissue with an EMR beam, patient data from a remote network in electronic communication with the treatment system, the patient data including at least one pretreatment image of the patient tissue exhibiting signs of a condition;
determining, by at least one processor, one or more recommended changes to a set of base treatment parameters of the EMR treatment device based on the received patient data;
offering the determined one or more recommended changes to a user of the system; and
appending the patient data with at least one posttreatment image of the patient tissue.

10. The system of claim 9, wherein the one or more recommended changes can include adjustments to laser pulse energy of the EMR beam, focus depth of the EMR beam, and energy density over the patient tissue.

11. The system of claim 9, wherein the determination of the one or more recommended changes uses a machine-learning algorithm.

12. The system of claim 9, further comprising comparing the at least one pretreatment image of the patient tissue and the at least one post treatment image of the patient tissue to grade progression of a treatment of the patient tissue with the EMR treatment device.

13. The system of claim 9, further comprising determining, from color analysis, a skin type of the patient tissue.

14. The system of claim 9, further comprising organizing and providing at least some of a stored patient data portfolio to create an individualized treatment plan.

15. The system of claim 9, further comprising authenticating access to the remote network prior to receiving the patient data therefrom.

16. The system of claim 15, wherein the authenticating is based at least partially on a verification of a current payment status.

17. A computer-readable medium storing instructions that, when executed by at least one processor, cause the processor to perform steps comprising:
receive, at a treatment system including an electromagnetic radiation (EMR) treatment device configured to treat patient tissue with an EMR beam, patient data from a remote network in electronic communication with the treatment system, the patient data including at least one pretreatment image of the patient tissue exhibiting signs of a condition;
determine one or more recommended changes to a set of base treatment parameters of the EMR treatment device based on the received patient data;
offer the determined one or more recommended changes; and
append the patient data with at least one posttreatment image of the patient tissue.
